# EUROPEAN PATENT APPLICATION

(11) **EP 4 059 955 A1**
(43) Date of publication of application: **21.09.2022**
(21) Application number: 21163630.3
(22) Date of filing: 19.03.2021
(51) Int. Cl.: C07K 14/725, A61K 35/17, A61P 35/00

(54) **HLA-DR-SPECIFIC GAMMA DELTA TCR CONSTRUCTS AND USE THEREOF**

(71) Applicant: Medizinische Hochschule Hannover, 30625 Hannover (DE)
(72) Inventor: Prinz, Immo, 22763 Hamburg (DE); Deseke, Malte, 30655 Hannover (DE)
(74) Representative: Moré, Solveig Helga

(57) **Abstract**

The present invention relates to the filed of immunotherapy, in particular, of lymphoproliferative disorders associated with abnormal proliferation of HLA-DR+ cells, e.g., malignancies of the hematopoietic and lymphoid tissues. The invention provides pharmaceutical compositions useful for, e.g., adoptive T cell therapy or T cell receptor (TCR) gene therapy or such disorders, as well as novel expression vectors, host cells and γδ (gamma/delta) TCR constructs. In particular, the inventors have identified γδ (gamma/delta) TCR constructs that can specifically bind to HLA-DR. In addition to host cells engineered to express such constructs that can be used for therapeutic as well as diagnostic purposes, soluble TCR constructs are provided, which can, e.g., be used in a method of detecting HLA-DR+ cells, e.g., in vitro as well as bispecific constructs that can be therapeutically used.

## Description

The present invention relates to the filed of immunotherapy, in particular, of lymphoproliferative disorders associated with abnormal proliferation of HLA-DR+ cells, e.g., malignancies of the hematopoietic and lymphoid tissues. The invention provides pharmaceutical compositions useful for, e.g., adoptive T cell therapy or T cell receptor (TCR) gene therapy or such disorders, as well as novel expression vectors, host cells and γδ (gamma/delta) TCR constructs. In particular, the inventors have identified γδ (gamma/delta) TCR constructs that can specifically bind to HLA-DR. In addition to expression vectors and host cells expressing such constructs that can be used for therapeutic as well as diagnostic purposes, soluble TCR constructs are provided, which can, e.g., be used in a method of detecting HLA-DR+ cells, e.g., *in vitro,* as well as bispecific constructs that can be therapeutically used.

Immunotherapy is becoming increasingly important in the fight against tumors. Properties of the immune system are exploited and specifically enhanced to eliminate tumor cells. One approach to this is the use of endogenous T cells, which can be specialized and redirected to enhance tumor targeting through transgenic expression of tumor-specific T cell receptors (abbreviated as TCRs) or CARs (abbreviation for Chimeric Antigen Receptors).

Specific immunotherapy can be based on transfer of CAR-T cells. The chimeric antigen receptors are recombinant molecules typically based on the variable, antigen-specific parts of antibodies, or alternatively, receptors or ligands of known receptor-ligand pairs, expressed as fusion proteins with intracellular signalling domains from TCRs and, typically of co-stimulatory molecules. Therefore, the use of CARs overcomes the problem of HLA-restriction, and CAR-T cells can be used in all patients expressing the tumor antigen for which the CAR is specific, without restriction to a particular HLA-type. One drawback of CAR T-cells however is that the cell surface expression of the antigen on the tumor cell is required for success of the therapy, and there is a high selection pressure on the tumor cells to downregulate said cell surface expression. Thus, while CAR-T cell therapies, e.g., with CD19-CAR-T cells against acute lymphoblastic leukemia, have been shown to be highly successful (Brudno *et al.,* 2020), in some patients, the tumor cells loose expression of CD19, and the therapy becomes ineffective after some time.

TCRs are heterodimers consisting of either an α- and a β-chain (αβ TCR) or a γ- and a δ-chain (γδ TCR). Each of these chains can be subdivided in a variable part, which is mediating the antigen binding and a membrane-anchored constant part interacting with the signal transducer CD3. The regions of the variable part determining the antigen specificity are called CDR (for complementarity-determining-region). CDR1 and CDR2 of α,β, γ- and δ-chains are germline-encoded and only vary between different V-fragments. The CDR3 however is composed by VDJ-recombination and therefore highly variable between different T cell clones.

By now, a high number of TCRs specific for tumor antigens are known, mostly αβ TCR. However, αβ TCRs almost exclusively recognize specific peptides presented by MHC I and II molecules, this is the so-called MHC restriction. Thus, an αβ TCR can only be therapeutically used in patients having a specific MHC or HLA allele, i.e., only in a fraction of all patients.

The use of αβ TCRs for immunotherapy thus requires knowledge of HLA-haplotype specificity in addition to the presented tumor-specific peptide. This limits the applicability to a certain subset of the population. γδ TCRs however, do not rely on different HLA-subtypes and could therefore be applied to a broader set of patients.

Ligands for γδ TCRs remain largely unknown. Based on the data on γδ TCR specificities identified so far, it is generally considered that the receptors bind to their targets in a way independent from MHC-presentation of peptides. They may bind to conformational epitopes, i.e. specific surface structures of proteins and possibly other molecules. For example, some γδ TCRs are known to bind to phosphoantigens and aminobisphosphonates that may be induced by metabolic changes in cells such as tumor cells. The target of a Vγ9Vδ2 TCR is known to be a joint spatial and conformational change of CD277 at the cell membrane of cancer cells. By contrast, ligands of non- Vγ9Vδ2 TCRs are poorly described, and may include endothelial protein C receptor, annexin A2, MHC class I-related chain A or B (MICA/B), different isoforms of CD1 and the classical HLA molecules (WO 2017212072 A1, Deseke and Prinz, 2020) or SCNN1A (US 20200316124 A1). In particular, certain varieties of γδ TCRs, namely those with a Vδ1 chain, seem to be able to recognize MHC or MHC-like molecules, and are at the same time known to react to tumor cells. Vδ1⁺ γδ TCRs are considered particularly suitable for anti-tumor immunotherapy, because they recognize specific proteins without relying on individual MHC variants (Flament *et al.,* 1994; Deseke and Prinz, 2020; Sebestyen *et al.,* 2020). Exact knowledge of the activating antigens is very important for this purpose to ensure specific and controlled activation. So far, very few γδ TCRs specific for known antigens expressed by tumor cells have been identified.

In light of the state of the art, the inventors solved the problem of providing an advantageous immunotherapy for a broad range of malignancies of the hematopoietic and lymphoid tissues, e.g., B cell lymphomas. The problem is solved by the subject-matter of the claims.

In particular, the invention provides a pharmaceutical composition comprising
a) a nucleic acid encoding a TCR gamma chain construct (TRG) and a TCR delta chain construct (TRD) of a TCR construct;
b) a TCR construct comprising a TRG and a TRD; and/or
c) a human lymphoid host cell comprising the nucleic acid(s) of a) and expressing the TCR construct of b);
wherein the TCR construct is specific for HLA-DR and wherein the TRD comprises a CDR3 having at least 95% sequence identity to SEQ ID NO: 1. The TRD preferably further comprises a CDR1 having at least 80% sequence identity to SEQ ID NO: 5 and a CDR2 having at least 66% sequence identity to QGS. The sequence identity of said CDR1 and CDR2 may also be 100%.

In the context of the invention, unless explicitly defined otehrwiese, "a" includes one or more, e.g., one or two. In particular, in the context of a nucleic acid encoding a TCR gamma chain construct (TRG) and a TCR delta chain construct (TRD) of a TCR construct, the skilled person understands that the TRG and the TRD can be encoded on one or two nucleic acids.

In the context of the present invention, the inventors identified two Vγ3Vδ1⁺ TCRs that recognize HLA-DR (MHC class II) with high affinity, TCR04 and TCR05. TCR constructs based on these γδ TCRs, nucleic acids encoding them or T cells expressing them can advantageously be used for immunotherapy, as the HLA-DR recognition is independent of the HLA-subtype and, at least for TCR04, also of the presented peptide. This allows for an application of the TCRs of the invention in fighting HLA-DR⁺ lymphomas and leukemias or other diseases and disorders caused by HLA-DR⁺ cells in a broad set of patients. Moreover, the reactivity is independent of the mutational load of the tumors, but solely requires the expression of HLA-DR.

In a study analyzing reconstitution of human γδ Tc ells after stem-cell transplantation, gamma and delta chains, seperately, had been sequenced, among them, the sequences of the delta chains of TCR04 and TCR05 (Ravens *et al.,* 2017). However, neither the paired gamma chains nor targets of the TCRs comprising said delta chains had been identified or suggested by said study. Therefore, only the present invention teaches and enables the application of the TCR constructs defined here, in particular, the therapeutic application.

The CDR3 regions of the delta chains of TCR04 and TCR05 differ in one amino acid only, which explains their similar functional characteristics. Thus, in the context of the invention, a TRD having a CDR3 with at least 95% sequence identity to SEQ ID NO: 1 may be employed. Compared to SEQ ID NO: 1, there is at most one amino acid exchange, deletion or insertion, typically, an amino acid exchange. The exchange may be a conserved substitution, i.e., one amino acid of a particular type (e.g., polar, apolar, acidic, basic, aromatic) is exchanged against another amino acid of the same type. Methods for affinity maturation may be employed. Such methods are, e.g., known in the art.

The TRD of the invention preferably comprises a CDR3 having SEQ ID NO: 2, wherein the amino acid differing between the CDR3 regions of TCR04 and TCR05 is variable (X). Preferably, in said TRD comprising a CDR3 having SEQ ID NO: 2, the amino acid in the variable position X is a hydrophobic amino acid, e.g., a non-aromatic hydrophobic amino acid (i.e., Val, Ala, Gly, Ile, Leu, Val or Met. Optionally, the amino acid in the variable position X is Val, Ala, Gly, Ile, Leu or Val, preferably Val, Gly or Ala.

In a preferred embodiment, the TRD of the invention comprises a CDR3 having SEQ ID NO: 1. This is the sequence of the CDR3 of TCR04, corresponding to SEQ ID NO: 2 wherein X is Val. The inventors have shown that this CDR3 endows the TCR construct with a very high affinity to HLA-DR.

Alternatively, the TRD comprises a CDR3 having SEQ ID NO: 3. This corresponds to the CDR3 of TCR05, which has a lower affinity, but a more narrow specificity enabling a more specific targeting. In particular, the inventors have shown that loading with peptide is essential for binding of TCR05, but not for binding of TCR04. In still another alternative, the TRD comprises a CDR3 having SEQ ID NO:4, wherein the variable amino acid is Ala. Said TRD may have intermediate characteristics with regard to affinity and specificity.

The TRD may, optionally, be a Vδ1D2J1 TRD.

The TRD may e.g. comprise a variable region having at least 80% sequence identity to SEQ ID NO: 7, optionally, at least 90% sequence identity, e.g., 100% sequence identity. The variable region of the TRD is optionally encoded by a nucleic acid having at least 80% sequence identity to SEQ ID NO: 8, optionally, at least 90% sequence identity, e.g., 100% sequence identity, but other codons may also be chosen. In particular, the nucleic acid may be codon-optimized for expression in a human cell.

All TCR constructs employed in the context of the invention are specific for HLA-DR. The terms "capable of specifically binding" or "recognizing" or "specific for" a given antigen, as used herein, are synonymous, and mean that the TCR construct can specifically bind to and immunologically recognize HLA-DR, preferably with high or, more preferably, very high affinity.

For example, a TCR construct may be considered to "be able of specifically binding" to HLA-DR if a corresponding soluble TCR construct shows significant binding to target cells expressing HLA-DR, e.g., Raji cells, which can be analysed by flow cytometric methods, wheras they do not show significant binding to cells not expressing HLA-DR, e.g., K562 cells.

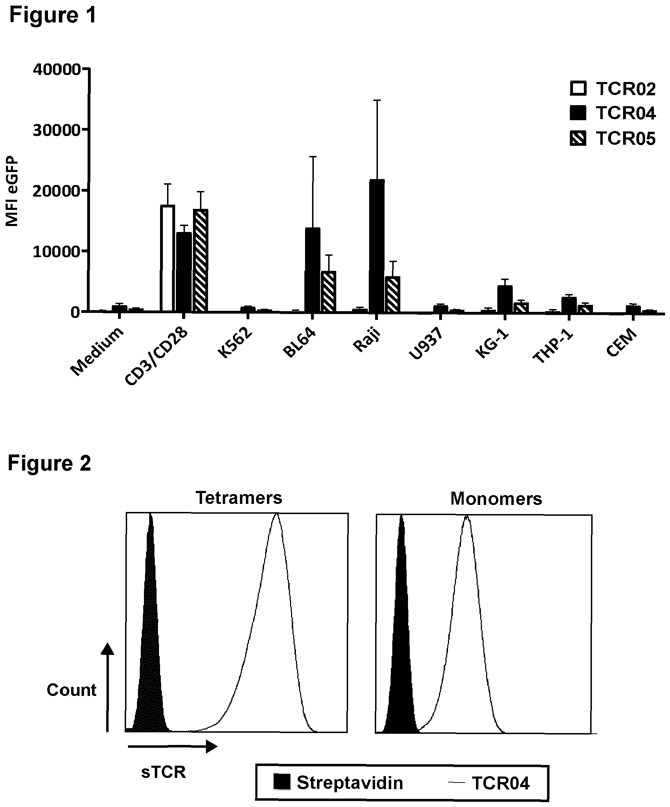

Activation of T cells expressing a TCR construct capable of specifically binding to HLA-DR can be tested by the methods disclosed in the examples below, e.g., as shown in Fig. 1.

Alternatively, or additionally, T cells expressing the TCR may secrete at least about 200 pg/mL or more (e.g. 250 pg/mL or more, 300 pg/mL or more, 400 pg/mL or more, 500 pg/mL or more, 600 pg/mL or more, 700 pg/mL or more, 1000 pg/mL or more, 2,000 pg/mL or more, 2,500 pg/mL or more, 5,000 pg/mL or more) of interferon γ (IFN-y) upon co-culture with target cells expressing HLA-DR, e.g., Raji cells, but not with cells not expressing HLA-DR, such as K562 cells. Preferably, this is tested with 10,000 TCR+ T cells and 20,000-50,000, preferably, 50,000 target cells expressing an appropriate HLA after overnight incubation. Alternatively, or additionally, a TCR may be considered to have "antigenic specificity" for HLA-DR if T cells expressing the TCR secrete at least twice as much IFN-γ as the untransduced background level of IFN-γ upon co-culture with target cells expressing HLA-DR, e.g., Raji cells. Such "specificity" as described above can - for example - be analyzed with an ELISA.

A high affinity is defined as an affinity that enables significant binding of tetrameric TCR constructs to HLA-DR+ cells, e.g., under the conditions in the experiment underlying Fig. 2. A very high affinity is defined as an affinity that enables significant binding of monomeric TCR constructs to HLA-DR+ cells, e.g., under the conditions in the experiment underlying Fig. 2. For example, TCR04 has a very high affinity.

Alternatively, affinity can be analyzed by methods well known to the skilled person, e.g. by BiaCore. A TCR affinity or T cell avidity of 100 µM or higher, more preferably 10 µM or higher is considered high affinity.

The inventors have shown that the delta chain is mainly responsible for binding to HLA-DR. While presence of a TRG is also essential for binding, the experiments herein demonstrate that the gamma chain can be exchanged without abolishing binding to HLA-DR, and in most cases, without even a significant loss of affinity. Preferably, therefor, the TRG is selected from the group consisting of a Vγ3 TRG, a Vγ5 TRG or a Vγ8 TRG. Most preferably the TRG is a Vγ3 TRG, e.g., a Vy3J1 TRG. The TRG optionally is not Vγ2, as said TRG was shown to reduce binding to HLA-DR.

Optionally, the TRG comprises a CDR3 having at least 80% sequence identity to SEQ ID NO: 10, such as at least 90%, e.g., 100% sequence identity. The TRG may also comprise a CDR1 having at least 80% sequence identity to SEQ ID NO: 11 and a CDR2 having at least 80% sequence identity to SEQ ID NO: 12, wherein, optionally, the sequence identities are 100%.

The TRG may e.g. comprise a variable region having at least 80% sequence identity to SEQ ID NO: 13, optionally, at least 90%, e.g., 100% sequence identity. The variable region of the TRG may be encoded by a nucleic acid having at least 80% sequence identity to SEQ ID NO: 14, optionally, at least 90%, e.g., 100% sequence identity. Other codons may also be chosen. In particular, the nucleic acid may be codon-optimized for expression in a human cell.

A TCR gamma and/or delta chain construct of the invention may comprise all characteristics or domains corresponding to its native counterpart, i.e., a TCR gamma or delta chain, but this is not essential. Preferably, the TCR gamma and/or delta chain construct comprises at least a variable region, or a variable and a constant region, e.g., the variable and/or constant region having at least 60%, at least 70%, at least 80%, at least 90% or at least 95% sequence identity to a human variable or constant TCR region.

The TCR gamma chain constructs and/or the TCR delta chain constructs of the invention preferably comprise a constant region. For adoptive TCR therapy, it is preferred that the TCR construct comprises full length TCR gamma or delta chains comprising variable and constant regions, including transmembrane regions and cytoplasmic regions. The constant region may be a human constant region, a murine constant region or a chimeric constant region such as a minimally murine constant region. The TCR construct may be of essentially or exclusively human origin to minimize immunogenicity. In addition, codon modification of the TCR sequence may be used to enhance the functional expression of the transgenic TCR (Scholten *et al.,* 2006) and/or a peptide (e.g. P2A) can be applied to link both TCR chains to achieve a stoichiometric expression of both chains (Leisegang *et al.,* 2008), also resulting in an enhanced functional expression of the transgenic TCR.

The construct may also be a chimeric antigen receptor (CAR), or part of it, wherein, e.g. a human TCR variable region may be linked to a different immunoglobulin constant domain, e.g. constant domains of αβ TCR, or to an IgG constant domain.

Single chain constructs (scTCR) are encompassed as well as heterodimeric TCR constructs. A scTCR can comprise a variable region of a first TCR chain construct (e.g., an gamma chain) and an entire (full-length) second TCR chain (e.g., a delta chain), or vice versa. Furthermore, the scTCR can optionally comprise one or more linkers which join the two or more polypeptides together. The linker can be, for instance, a peptide which joins together two single chains. Also provided is such a scTCR of the invention, which is fused to a cytokine, e.g., a human cytokine, such as IL-2, IL-7, IL-12 or IL-15.

In one embodiment, the pharmaceutical composition of the invention comprises a) one or two nucleic acid(s) encoding a TCR gamma chain construct (TRG) and a TCR delta chain construct (TRD) of a TCR construct. To enable specific recognition of HLA-DR, in particular, for therapeutic or diagnostic purposes, the nucleic acid(s) of the invention encode(s) one TCR gamma and one delta chain construct of a TCR construct of the invention, as described herein.

As the TCR construct of the invention, when employed for pharmaceutical purposes, contains both gamma and delta chain constructs, it may be encoded by either one or two nucleic acids. In the context of the invention, if reference is made to a nucleic acid, this also applies to two nucleic acids. The TRG and TRD together are specific for HLA-DR, i.e., capable of specifically binding to HLA-DR. The invention also provides a single chain nucleic acid construct, wherein, e.g., TCR gamma and delta chain constructs are separated by a P2A element.

The sequences encoding the TRG and/or TRD are functionally linked to a promotor operable in human cells. Thus, the nucleic acid is an expression vector suitable for expression in human cells, e.g., human lymphoid cells. It can e.g. be used for gene therapy of a human patient in need thereof.

Alternatively, the pharmaceutical composition of the invention comprises b) a TCR construct comprising a TRG and a TRD. The TCR construct is thus also suitable for administration in protein form. For example, a monomeric or multimeric (e.g., tetrameric) TCR construct of the invention can be for use in targeting a cell, a compound, a virus or, e.g., a vesicle to a cell expressing HLA-DR such as a tumor cell. The vesicle, e.g., enveloped by a lipid membrane, may comprise a compound. The compound may be a therapeutic compound, e.g., a toxic compound, or a prodrug of a toxic compound. With the aid of a TCR construct of the invention, a cell expressing HLA-DR may also be marked or labelled, e.g., for targeting with another agent.

In a preferred embodiment, the pharmaceutical composition of the invention comprises c) a human lymphoid host cell comprising the nucleic acid(s) of a) and expressing the TCR construct of b), e.g., a T cell, NK cell or NK/T cell. Such cells may advantageouly be used for adoptive T cell therapy or a patient, e.g., as further described herein.

In another embodiment, the invention provides a **non-wild-type TCR construct,** namely, a TCR construct comprising a TRG and a TRD, wherein the TCR construct is specific for HLA-DR and wherein the TRD comprises a CDR3 having at least 95% sequence identity to SEQ ID NO: 1, and wherein the TCR construct is
a) a soluble TCR construct and/or
b) a bispecific TCR construct and/or
c) a chimeric TCR construct and/or
d) a scTCR construct.

Said protein constructs may be employed, e.g., for targeting cells expressing HLA-DR, e.g.,. for diagnostic or therapeutic purposes. For example, a monomeric or multimeric (e.g., tetrameric) TCR construct of the invention can be for use in targeting a cell, a compound or agent, a virus or, e.g., a vesicle or a bacterial minicell to a cell expressing HLA-DR such as a tumor cell. The vesicle, e.g., enveloped by a lipid membrane, may comprise a compound. The compound or agent may be a therapeutic compound, e.g., a toxic compound, or a prodrug of a toxic compound. With the aid of a TCR construct of the invention, a cell expressing HLA-DR may also be marked or labelled, e.g., for targeting with another agent. The compound may also be a label, such as a fluorescent label or radioactive label, e.g., for diagnostic purposes. The TCR construct of the invention may be for use *in vivo,* or it may be used *in vitro.* For example, a monomeric or multimeric, e.g., tetrameric soluble TCR construct of the invention may be used for labelling, e.g., sorting cells expressing HLA-DR, e.g., in a FACS or ELISPOT assay.

A soluble TCR construct typically lacks transmembrane regions. It may be a fusion protein, e.g., comprising an Fc part, such as an IgG Fc part (or, e.g., an IgM or IgA Fc part). An exemplary soluble TRD is provided in SEQ ID NO: 17, and a soluble TRG construct is provided in SEQ ID NO: 18. As in said sequences, a soluble TCR construct may comprise a tag e.g., for multimerisation, such as a Twin-Strep-Tag. Of course, a soluble TCR may also be expressed without such a tag. A soluble TCR construct, or the chains thereof may also comprise a signal peptide for extracellular expression, but that is not required, because, e.g., the protein can be obtained from intracellular expression, or a signal peptide may be cleaved off. For example, SEQ ID NO: 17 and 18 further comprise a signal peptide for expression in insect cells, which may be emploed. Signal peptides for expression in human cells may alternatively be used. A soluble TCR construct having the sequences of SEQ ID NO: 17 and 18 without the signal peptide and/or without the tag are also disclosed herewith.

A bispecific TCR construct of the invention may, e.g., be a fusion protein comprising an scTCR of the invention in combination with another scTCR. The second specificity of a bispecific TCR of the invention may be directed to, e.g., a surface antigen expressed by HLA-DR positive cells, e.g., on B-cells, macrophages, dendritic cells, activated T-cells, etc., either exclusively on one of said cell types or on several of them. If the second target is only expressed on one of these cell types, this will increase specificity of the construct for said cell type. For example, it may be CD19, or an Fc receptor such as CD16, CD32 or CD64.

Alternatively, if the bispecific construct is soluble, the second specificity may be directed to a surface antigen of a T cell, e.g.,CD3 (Robinson *et al.,* 2021), or to a Vγ9Vδ2-TCR , or the bispecific TCR may, in one specificity, be a Vγ9Vδ2-TCR construct (Oberg *et al.,* 2014, 2015, 2020; de Bruin *et al.,* 2018).

Optionally, said bispecific TCR may further be a fusion protein comprising a human or non-human Fc part of an immunoglobuloin, e.g., of IgG (or, e.g., an IgM or IgA Fc part). A bispecific TCR construct of the invention has at least two specificities. It may also have more, e.g., three, four or more specificities (Oberg *et al.,* 2015). That may also be achieved by multimerization, e.g., via biotin-steptavidin or biotin-avidin binding. A bispecific TCR construct may be soluble, or it may comprise a transmembrane region. It may be expressed by a lymphoid cell such as a T cell. A bispecific TCR construct may be chimeric or of fully human origin.

A chimeric TCR construct of the invention has at least two parts derived from different species, e.g., it may comprise, in addition to a human variable region, a murine or partially murine (e.g., minimally murine) constant region. A chimeric TCR construct may be soluble, or it may comprise a transmembrane and cytoplasmic region, e.g., it may be expressed by a lymphoid cell such as a T cell. A chimeric TCR construct may also be bispecific or not bispecific.

The TCR construct of the invention may also be a scTCR, e.g., a soluble or transmembrane scTCR. It may be a bispecific TCR construct, and/or a chimeric TCR construct.

Throughout the invention, in protein form, the TRG and the TRD preferably form a soluble heterodimer, optionally, associated with a further agent such as a fluorescent label, a tag, and a lipidic moiety.

TCR constructs of the invention may be non-glycosylated (e.g., when used in soluble protein form) or, preferably, glycosylated. Preferably, the glycosylation pattern corresponds to the pattern of human gamma delta TCR, but e.g. glycosylation obtained in insect cells also leads to a functional TCR.

The present invention also provides an **expression construct,** in particular, an expression vector encoding a TCR gamma chain construct (TRG) and a TCR delta chain construct (TRD) of a TCR construct specific for HLA-DR, wherein the TRD comprises a CDR3 having at least 95% sequence identity to SEQ ID NO: 1, wherein expression or the TRG and/or TRD is controlled by a heterologous promotor capable of mediating expression in a lymphoid cell. The TRD preferably further comprises a CDR1 having at least 80% sequence identity to SEQ ID NO: 5 and a CDR2 having at least 66% sequence identity to QGS. The sequence identity of said CDR1 and CDR2 may also be 100%. The TCR construct may have the characteristics as defined herein, e.g., above, in reference to the pharmaceutical composition or a non-wildtype TCR construct.

The present invention also provides an expression construct, in particular, an expression vector encoding a TCR gamma chain construct (TRG) and a TCR delta chain construct (TRD) of a TCR construct specific for HLA-DR, wherein the TCR construct is a non-wildtype TCR construct as described above, e.g.,
a) a soluble TCR construct and/or
b) a bispecific TCR construct and/or
c) a chimeric TCR construct and/or
d) a scTCR construct.

Of course, expression or the TRG and/or TRD of said expression constructs may also be controlled by a heterologous promotor capable of mediating expression in a lymphoid cell.

In the context of an expression construct of the invention, the nucleic acid can either be DNA or RNA. Preferably, it is DNA. The nucleic acid may, e.g., be a viral vector, or a non-viral vector, e.g., a transposon, a vector suitable for CRISPR/CAS based recombination or a plasmid suitable *for in vitro* RNA transcription. The nucleic acid of the invention preferably is a vector. Suitable vectors include those designed for expression, or for both expression and propagation and expansion, such as plasmids and viruses. The vector preferably is an expression vector suitable for expression in a human host cell, in particular, a human lymphoid host cell.

The vector may be a viral vector, e.g. a retroviral, in particular gamma-retroviral or lentiviral vector. Examples of suitable expression vectors include the retroviral vector MP71 (Engels *et al.,* 2003). The expression vector comprises regulatory sequences, such as transcription and translation initiation and termination codons, which are specific to the type of host cell into which the vector is to be introduced and in which the expression of the nucleic acid of the invention shall be performed, typically, in the context of the invention, human lymphoid cells such as T cells. Furthermore, the vector of the invention may include one or more marker genes, which allows for selection of transduced or transfected hosts. The recombinant expression vector can comprise a native or, preferably, a heterologous promoter operably linked to the nucleotide sequence encoding the TCR construct of the invention, or to a nucleotide sequence which is complementary to or which hybridizes to the nucleotide sequence encoding the constructs of the invention. The selection of promoters includes, e.g., strong, weak, inducible, tissue-specific and developmental-specific promoters. The promoter can be a non-viral promoter or a viral promoter. Preferably, it is a heterologous promoter, i.e., a promoter not naturally linked to TCR in human T cells, such as long terminal repeat promoter, which is suitable for expression in human T cells. The inventive recombinant expression vectors can be designed for either transient expression, for stable expression, or for both. Also, the recombinant expression vectors can be made for constitutive expression or for inducible expression.

The invention further provides a **host cell** comprising the expression vector of the invention. Said host cell preferably is a human lymphoid host cell, wherein the cell is selected from the group comprising a T cell, an NK cell and an NK/T cell or a T cell precursor.

Generally, in the context of the invention, the lymphoid host cells is a human cell selected from the group comprising a T cell or an NK cell. Preferably, it is a human T cell, such as CD4+ or CD8+ T cell. It may also be a γδT cell. Rather, αβ T cells expressing a γδTOP (so-called TEG, T cells engineered with defined γδTCRs) may combine the advantages of γδ TCRs with the high proliferation and memory capacitiy of αβ T cells. A mixture of αβ and γδ T cells may also be used. If both CD4+ and CD8+ T cells are engineered to express the γδ TCR construct, not only are cytotoxic properties present, but CD4+ TEGs also have the ability to mature professional antigen-presenting cells such as DC (Sebestyen *et al.,* 2020). NK cells are also suitable for use as host cells. For example, with NK cells, there are no problems with expression of native TCRs. As shown by Mensali *et al.,* 2019, NK cells such as the NK cell line NK-92 can express and use TCR, co-expressed with CD3. Alternatively, αβ TCR genes can be removed from αβ T cells via CRISPR technology prior to engineering to express the γδ TCR construct, or miRNA can be used to suppress expression of the αβ TCR.

The T cell may be a central-memory T cell, effector-memory T cell, stem cell-like T cell or effector T cell, or a mixture of these.

The host cell may further express co-receptors and/or signalling molecules such as NKG2D, DNAM1, NKp30, NKp44, CD4 and/or CD3, but that is not required.

The host cell preferably is a peripheral blood leukocyte (PBL) or a peripheral blood mononuclear cell (PBMC). It may be human T cell isolated from PBMC. The T cell can however be any T cell, such as a cultured T cell, e.g. a primary T cell, or a T cell from a cultured T cell line. The T cell can be obtained from numerous sources, such as blood, bone marrow, lymph node, the thymus, or other tissues or fluids. T cells can also be enriched for or purified. It can be, e.g., a tumor infiltrating cell (TIL), effector cell, central effector cell, memory T cell, naive T cell, and the like, preferably a central-memory T cell. Alternatively, the host cell may be another immune effector cell, e.g., an NK cell or an NK/T cell.

Preferably, the host cell is a cell isolated from a human, e.g., a human patient, in particular, the patient who is to be treated. Alternatively, the T cell may be from a third-party donor who may be related or unrelated to the patient. Such T cells may be genetically engineered, e.g., in multiple ways. For example, by knock out of the endogenous TCR and/or MHC I. T cells may also be generated from autologous or third-party donor stem cells, wherein, optionally, the stem cells are not human embryonic stem cells.

Optionally, host cells of the invention may be engineered by reducing expression of MHC II, e.g., by knocking out or knocking down HLA-DR, for example through Cas9-mediated recombination. As human activated T cells may express HLA-DR, this may prevent fratricide of the transgenic T cells. Alternatively, depending on the strength of the desired immune response, a self-limitation can be considered beneficial, e.g., to avoid undesired effects of the anti-HLA-DR response of the transferred T cells.

Preferably, the host cell is a human NK cell or T cell, e.g., an αβ or γδT cell, comprising a nucleic acid of the invention which is an expression vector, wherein the nucleic acid encoding the TRC and/or TRD is operably linked to a heterologous promotor, and wherein the host cell expresses a TCR construct as defined herein. As explained above, use of T cells, in particular αβ T cells may be preferred.

The invention also provides a host cell of the invention expressing two or more different TCR constructs. Preferably, in this context, the TCR constructs are single chain TCR constructs in which the TCR delta and gamma chains of at least one TCR are combined by a linker to avoid mispairing between transgenic TCR chains. The cells may also express a gamma/delta TCR construct of the invention and an alpha/beta TCR construct. Two TCR constructs, e.g., two single chain TCR constructs may be encoded on a single expression vector.

The **pharmaceutical composition** of the invention may advantegously be for use in treatment of a human patient having a lymphoproliferatve disorder associated with abnormal proliferation of HLA-DR+ cells. The invention thus also discloses a method of treating of a human patient having a lymphoproliferatve disorder associated with abnormal proliferation of HLA-DR+ cells.

Constitutive expression of HLA-DR is restricted to the surfaces of three distinct types of antigen-presenting cells: dendritic cells, monocyte/macrophage lineage, and B lymphocytes. In human subjects, activated T cells express MHC class II as well. Most other cell types do not usually express MHC class II molecules, but such expression can be induced by a variety of stimuli, of which the most potent and known is IFN-γ. The cells most relevant for therapeutic targeting are malignant cells of the hematopoietic and lymphoid tissues expressing HLA-DR, e.g., B-cell lymphomas.

For example, the pharmaceutical composition of the invention may be for use in treating a malignancy of the hematopoietic and lymphoid tissues such as lymphoma, e.g., Hodgkin's lymphoma or Non-Hodgkin's lymphoma, e.g., Burkitt's lymphoma, leukemia, e.g., acute lymphoblastic leukemia (ALL), acute myelogenous leukemia (AML), chronic lymphocytic leukemia (CLL), chronic myelogenous leukemia (CML), or acute monocytic leukemia (AMoL), myeloma, e.g., multiple myeloma, and follicular dendritic cell sarcoma. HLA-DR expression of the tumor cells can be tested before treatment, e.g., with a soluble TCR construct of the invention.

One of the advantages of the present invention is that the patient does not have to express a specific HLA-DR allele. As shown herein, not even expression of a peptide on the HLA-DR is required for binding of TCR constructs comprising SEQ ID NO: 1, while absence of a peptide appears to reduce binding to HLA-DR for TCR constructs comprising SEQ ID NO: 3.

The treatment typically is an immunotherapy selected from the group comprising adoptive T cell therapy, TCR gene therapy, or a treatment involving targeting of HLA-DR+ cells with the TCR construct in soluble form or as a bispecific antibody. For adoptive T cell therapy, human lymphoid cells expressing the TCR construct, as described herein, are to be administered to the patient, in particular, an effective amount thereof. Suitable treatment schemes are, e.g., known in the art.

T cells are typically administered to a patient in a concentration of 1 x 10⁵ - 1 x 10⁹ cells per kg. About 1 x 10⁶ - 1 x 10¹¹ cells may be administered to a patient as a single dose. These parameters may be adapted by the medical practioner, depending, e.g., on the patients age, sex, body weight and medical condition. Protocols disclosed (e.g. in Doran *et al.,* 2019) may be adapted to use the host cells of the present invention. A lymphoablative treatment may be administered before, e.g., as known in the art.

Optionally, the treatment is TCR gene therapy. Alternatively, the treatment may involve targeting of HLA-DR+ cells with the TCR construct in soluble form. The treatment may also involve targeting of HLA-DR+ cells with the TCR construct as a bispecific antibody.

The invention also provides a method of detecting or targeting a HLA-DR+ cell, either *in vivo* or *in vitro.* Said method may comprise contacting cells (typcially, a population of cells comprising HLA-DR+ cells) with a host cell comprising the expression vector of the invention and expressing the TCR construct as disclosed herein, or with the TCR construct disclosed herein, in particular, said soluble, bispecific and/or TCR construct, wherein saif TCR construct is optionally labelled. This may be carried out as an *in vitro* method comprising contacting a sample from a human with said TCR construct or said host cell.Said method may be used, e.g., for diagnostic purposes, in particular, for quantifying HLA-DR+ cells, or for diagnosing a malignancy of the hematopoietic and lymphoid tissues selected from the group comprising lymphoma, e.g., Hodgkin's lymphoma or Non-Hodgkin's lymphoma, leukemia, e.g., acute lymphoblastic leukemia (ALL), acute myelogenous leukemia (AML), chronic lymphocytic leukemia (CLL), chronic myelogenous leukemia (CML), or acute monocytic leukemia (AMoL), myeloma, e.g., multiple myeloma, and follicular dendritic cell sarcoma

The pharmaceutical compositions or kits of the invention typically are for intravenous administration. They may further comprise pharmaceutically acceptable carriers such as buffers, e.g., physiological saline or PBS. They may further comprise exciptients, such as stabilizers such as SPGA, carbohydrates (e.g. sorbitol, mannitol, starch, sucrose, glucose, dextran), proteins such as albumin or casein, or protein containing agents such as bovine serum or skimmed milk.

The present invention is further illustrated in the following embodiments and examples with reference to the accompanying figures and sequences, nevertheless, without being limited thereto. For the purposes of the present invention, all references as cited herein are incorporated by reference in their entirety.

### The invention provides, e.g., the following embodiments:

As embodiment 1, the invention provides a pharmaceutical composition comprising
a) a nucleic acid (e.g., one or two nucleic acid(s) encoding a TCR gamma chain construct (TRG) and a TCR delta chain construct (TRD) of a TCR construct;
b) a TCR construct comprising a TRG and a TRD; and/or
c) a human lymphoid host cell comprising the nucleic acid(s) of a) and expressing the TCR construct of b);
wherein the TCR construct is specific for HLA-DR and wherein the TRD comprises a CDR3 having at least 95% sequence identity to SEQ ID NO: 1.

In embodiment 2, the pharmaceutical composition of embodiment 1 comprises one or two nucleic acid(s) encoding a TCR gamma chain construct (TRG) and a TCR delta chain construct (TRD) of a TCR construct. In embodiment 3, the pharmaceutical composition of embodiment 1 comprises TCR construct comprising a TRG and a TRD. In embodiment 4, the pharmaceutical composition of embodiment 1 comprises a human lymphoid host cell comprising the nucleic acid(s) of a) and expressing the TCR construct of b);
As embodiment 5, the invention provides an expression vector encoding a TCR gamma chain construct (TRG) and a TCR delta chain construct (TRD) of a TCR construct specific for HLA-DR, wherein the TRD comprises a CDR3 having at least 95% sequence identity to SEQ ID NO: 1,
i) wherein expression or the TRG and/or TRD is controlled by a heterologous promotor capable of mediating expression in a lymphoid cell, and/or
ii) wherein the TCR construct is
   a) a soluble TCR construct and/or
   b) a bispecific TCR construct and/or
   c) a chimeric TCR construct and/or
   d) a scTCR construct
   preferably, i).

As embodiment 6, the invention provides a host cell comprising the expression vector of embodiment 5. In embodiment 7, the host cell of embodiment 6 is a human lymphoid host cell. In embodiment 8, the host cell of any of embodiments 5-7 is selected from the group consisting of a T cell, an NK cell and an NK/T cell. In embodiment 9, the lymphoid host cell of embodiment 8 is a T cell such as an αβ T cell. In embodiment 10, the lymphoid host cell of embodiment 8 is a γδ T cell.

As embodiment 11, the invention provides a TCR construct comprising a TRG and a TRD, wherein the TCR construct is specific for HLA-DR and wherein the TRD comprises a CDR3 having at least 95% sequence identity to SEQ ID NO: 1, and wherein the TCR construct is
a) a soluble TCR construct and/or
b) a bispecific TCR construct and/or
c) a chimeric TCR construct and/or
d) a scTCR construct.

In embodiment 12, the TCR construct of embodiment 11 is a soluble TCR construct. In embodiment 13, the TCR construct of embodiment 11 is a bispecific TCR construct. In embodiment 14, the TCR construct of embodiment 11 is a chimeric TCR construct.

In embodiment 15, the pharmaceutical composition of any of embodiments 1-4 comprises the expression vector of any of embodiments 5 or 7-10, the host cell of any of embodiments 6-10 or the TCR construct of any of embodiments 11-14.

In embodiment 16, in the pharmaceutical composition of any of embodiments 1-4 or 15, the expression vector of any of embodiments 5 or 7-10, the host cell of any of embodiments 6-10 or the TCR construct of any of embodiments 11-14, the TRD comprises a CDR3 having SEQ ID NO: 2.

In embodiment 17, in the TRD of embodiment 16 comprising a CDR3 having SEQ ID NO: 2, the amino acid in the variable position X is a hydrophobic amino acid, preferably, a non-aromatic hydrophobic amino acid (i.e., Val, Ala, Gly, Ile, Leu, Val or Met. In embodiment 18, in the TRD of embodiment 17 comprising a CDR3 having SEQ ID NO: 2, the amino acid in the variable position X is Val, Ala, Gly, Ile, Leu or Val, preferably Val, Gly or Ala.

In embodiment 19, in the pharmaceutical composition of any of embodiments 1-4 or 15-18, the expression vector of any of embodiments 5 or 7-10 or 16-18, the host cell of any of embodiments 6-10 or 16-18 or the TCR construct of any of embodiments 11-14 or 16-18, the TRD comprises a CDR3 having SEQ ID NO: 1.

In embodiment 20, in the pharmaceutical composition of any of embodiments 1-4 or 15-18, the expression vector of any of embodiments 5 or 7-10 or 16-18, the host cell of any of embodiments 6-10 or 16-18 or the TCR construct of any of embodiments 11-14 or 16-18, the TRD comprises a CDR3 having SEQ ID NO: 3. In embodiment 21, in the pharmaceutical composition of any of embodiments 1-4 or 15-18, the expression vector of any of embodiments 5 or 7-10 or 16-18, the host cell of any of embodiments 6-10 or 16-18 or the TCR construct of any of embodiments 11-14 or 16-18, the TRD comprises a CDR3 having SEQ ID NO:4.

In embodiment 22, in the pharmaceutical composition of any of embodiments 1-4 or 15-21, the expression vector of any of embodiments 5 or 7-10 or 16-21, the host cell of any of embodiments 6-10 or 16-21 or the TCR construct of any of embodiments 11-14 or 16-21, the TRD is a Vδ1D2J1 TRD.

In embodiment 23, in the pharmaceutical composition of any of embodiments 1-4 or 15-22, the expression vector of any of embodiments 5 or 7-10 or 16-22, the host cell of any of embodiments 6-10 or 16-22 or the TCR construct of any of embodiments 11-14 or 16-22, the TRD comprises a CDR1 having at least 80% sequence identity to SEQ ID NO: 5 and a CDR2 having at least 66% sequence identity to QGS. In embodiment 24, the sequence identity of the CDR2 and CDR3 in embodiment 23 is 100%.

In embodiment 25, in the pharmaceutical composition of any of embodiments 1-4 or 15-24, the expression vector of any of embodiments 5 or 7-10 or 16-24, the host cell of any of embodiments 6-10 or 16-24 or the TCR construct of any of embodiments 11-14 or 16-24, the TRD comprises a variable region having at least 80% sequence identity to SEQ ID NO: 7, optionally, at least 90% sequence identity, e.g., 100% sequence identity. In embodiment 26, in the pharmaceutical composition of any of embodiments 1-4 or 15-25, the expression vector of any of embodiments 5 or 7-10 or 16-25, the host cell of any of embodiments 6-10 or 16-25 or the TCR construct of any of embodiments 11-14 or 16-25, the variable region of the TRD is encoded by a nucleic acid having at least 80% sequence identity to SEQ ID NO: 8, optionally, at least 90% sequence identity, e.g., 100% sequence identity.

In embodiment 27, in the pharmaceutical composition of any of embodiments 1-4 or 15-26, the expression vector of any of embodiments 5 or 7-10 or 16-26, the host cell of any of embodiments 6-10 or 16-26 or the TCR construct of any of embodiments 11-14 or 16-26, the TRG is selected from the group consisting of a Vγ3 TRG, a Vγ5 TRG or a Vγ8 TRG, preferably a Vγ3 TRG. The TRG optionally is not Vγ2. In embodiment 28, in the pharmaceutical composition of any of embodiments 1-4 or 15-27, the expression vector of any of embodiments 5 or 7-10 or 16-27, the host cell of any of embodiments 6-10 or 16-27 or the TCR construct of any of embodiments 11-14 or 16-27, the TRG is a Vγ3 TRG, preferably, a Vy3J1 TRG.

In embodiment 29, in the pharmaceutical composition of any of embodiments 1-4 or 15-28, the expression vector of any of embodiments 5 or 7-10 or 16-28, the host cell of any of embodiments 6-10 or 16-28 or the TCR construct of any of embodiments 11-14 or 16-28, the TRG comprises a CDR3 having at least 80% sequence identity to SEQ ID NO: 10, optionally, at least 90%, e.g., 100% sequence identity.

In embodiment 30, in the pharmaceutical composition of any of embodiments 1-4 or 15-29, the expression vector of any of embodiments 5 or 7-10 or 16-29, the host cell of any of embodiments 6-10 or 16-29 or the TCR construct of any of embodiments 11-14 or 16-29, the TRG comprises a CDR1 having at least 80% sequence identity to SEQ ID NO: 11 and a CDR2 having at least 80% sequence identity to SEQ ID NO: 12, wherein, optionally, the sequence identities are 100%.

In embodiment 31, in the pharmaceutical composition of any of embodiments 1-4 or 15-30, the expression vector of any of embodiments 5 or 7-10 or 16-30, the host cell of any of embodiments 6-10 or 16-30 or the TCR construct of any of embodiments 11-14 or 16-30, the TRG comprises a variable region having at least 80% sequence identity to SEQ ID NO: 13, optionally, at least 90%, e.g., 100% sequence identity. The variable region of the TRG may be encoded by a nucleic acid having at least 80% sequence identity to SEQ ID NO: 14, optionally, at least 90%, e.g., 100% sequence identity.

In embodiment 32, in the pharmaceutical composition of any of embodiments 1-4 or 15-31, the expression vector of any of embodiments 5 or 7-10 or 16-31, the host cell of any of embodiments 6-10 or 16-31 or the TCR construct of any of embodiments 11-14 or 16-31, the TRG and/or the TRD further comprise a constant region selected from the group comprising a human constant region, a murine constant region or a chimeric constant region.

In embodiment 33, in the pharmaceutical composition of any of embodiments 1-4 or 15-32, the expression vector of any of embodiments 5 or 7-10 or 16-32, the host cell of any of embodiments 6-10 or 16-32 or the TCR construct of any of embodiments 11-14 or 16-32, the TRG and the TRD form a soluble heterodimer, optionally, associated with a further agent selected from the group comprising a fluorescent label, a tag, and a lipidic moiety.

In embodiment 34, in the pharmaceutical composition of any of embodiments 1-4 or 15-33, the expression vector of any of embodiments 5 or 7-10 or 16-33, the host cell of any of embodiments 6-10 or 16-33 or the TCR construct of any of embodiments 11-14 or 16-33, the nucleic acid is selected from the group consting of a viral vector, a transposon, a vector suitable for CRISPR/CAS based recombination or a plasmid suitable for *in vitro* RNA transcription, or wherein the nucleic acid is DNA integrated into the genomic DNA of a host cell.

In embodiment 35, in the pharmaceutical composition of any of embodiments 1-4 or 15-34, the expression vector of any of embodiments 5 or 7-10 or 16-34, the host cell of any of embodiments 6-10 or 16-34 or the TCR construct of any of embodiments 11-14 or 16-34, the host cell is a human lymphoid host cell selected from the group comprising a T cell, NK cell or NK/T cell. In embodiment 36, the lymphoid host cell of embodiment 35 is a T cell such as an αβ T cell. In embodiment 37, the lymphoid host cell of embodiment 35 is a γδ T cell. In embodiment 38, the lymphoid host cell of embodiment 35 is an NK cell. In embodiment 39, the lymphoid host cell of embodiment 35 is a NK/T cell.

In embodiment 40, the invention provides the pharmaceutical composition of any of embodiments 1-4 or 15-39 for use in treatment of a human patient having a lymphoproliferatve disorder associated with abnormal proliferation of HLA-DR+ cells. In embodiment 41, the pharmaceutical composition for use of embodiment 141 is for use in treating a malignancy of the hematopoietic and lymphoid tissues selected from the group comprising lymphoma, e.g., Hodgkin's lymphoma or Non-Hodgkin's lymphoma, leukemia, e.g., acute lymphoblastic leukemia (ALL), acute myelogenous leukemia (AML), chronic lymphocytic leukemia (CLL), chronic myelogenous leukemia (CML), or acute monocytic leukemia (AMoL), myeloma, e.g., multiple myeloma, and follicular dendritic cell sarcoma.

In embodiment 42, with regard to the pharmaceutical composition for use of any of embodiments 40-41, the treatment is an immunotherapy selected from the group comprising adoptive T cell therapy, TCR gene therapy, or a treatment involving targeting of HLA-DR+ cells with the TCR construct in soluble form or as a bispecific antibody. In embodiment 43, with regard to the pharmaceutical composition for use of any of embodiments 40-42, the treatment is adoptive T cell therapy. In embodiment 44, with regard to the pharmaceutical composition for use of any of embodiments 40-42, the treatment is TCR gene therapy. In embodiment 45, with regard to the pharmaceutical composition for use of any of embodiments 40-42, the treatment involves targeting of HLA-DR+ cells with the TCR construct in soluble form. In embodiment 46, with regard to the pharmaceutical composition for use of any of embodiments 40-42 or 45, the treatment involves targeting of HLA-DR+ cells with the TCR construct as a bispecific antibody.

In embodiment 47, the invention provides a method of detecting or targeting a HLA-DR+ cell, comprising contacting said cell with the host cell of any of embodiments 6-10 or 16-34 or the TCR construct of any of embodiments 11-14 or 16-34, optionally, said TCR construct. In embodiment 48, the method of embodiment 47 is an *in vitro* method comprising contacting a sample from a human with said TCR construct or said host cell.

### Legends

**Fig. 1****: Results of co-culture experiments of γδ TCR expressing JE6.1 reporter cells with different leukemia cell lines as targets.** Cells were put together in a ratio of 1:1 and cultured overnight. The TCR-specific activation was detected as GFP fluorescence via flow cytometry. As a negative control, the reporter cells were cultured in medium. For positive control, TCR signaling was stimulated with CD3 and CD28 stimulation via antibodies. K562: CML line; BL64: Burkitt's lymphoma (B cell); Raji: Burkitt's lymphoma (B cell); U937: monocytic lymphoma; KG-1: monocytic cell line; THP-1: AML cell line; CEM: T cell lymphoblastic leukemia cell line. MFI: Mean fluorescence intensity.
**Fig. 2****: Soluble TCR staining of Raji cells with TCR04.** The soluble TCR was either tetramerized prior to the staining via streptavidin-PE or was used as monomers with a subsequent secondary detection via streptavidin-PE. As a negative control, cells were treated with just streptavidin-PE.

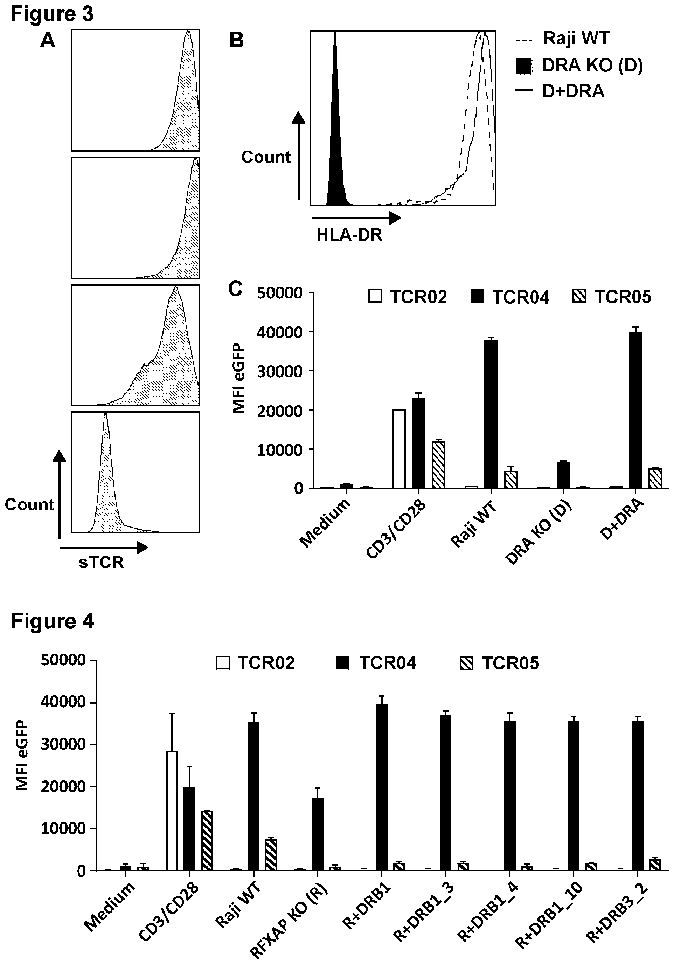

**Fig. 3****: Identification of HLA-DR as a ligand for TCR04 and TCR05.** A: Sorting of Raji cells transduced with a Genome-wide CRISPR-Cas9 Knock out library. The cells were sorted four times after staining with sTCR04 to accumulate cells that lost sTCR binding. B: Comparison of HLA-DR expression between different Raji cell lines. The sgRNA targeting the HLA-DRα chain along with Cas9 was introduced into Raji cells for verification (DRA KO). To rescue the TCR reactivity, the HLA-DRα chain was re-expressed in the DRA KO background (D + DRA). C: The described Raji cell lines were co-cultured with the reporter cells expressing TCR02, TCR04 and TCR05 overnight. MFI: Mean fluorescence intensity.
**Fig. 4****: Test for the influence of the HLA-DR haplotype on reactivity of TCR04 and TCR05.** In the RFXAP Knock out Raji cell line (RFXAP KO), different combinations of the invariant HLA-DRα chain with a subset of β-chain sequences were expressed (e.g. R + DRB1). For simplicity reasons, only the β-chain name is indicated. The reporter cells were co-cultured with the Raji cell lines at a ratio of 1:1 overnight and the GFP fluorescence as read-out was detected via flow cytometry. MFI: Mean fluorescence intensity.

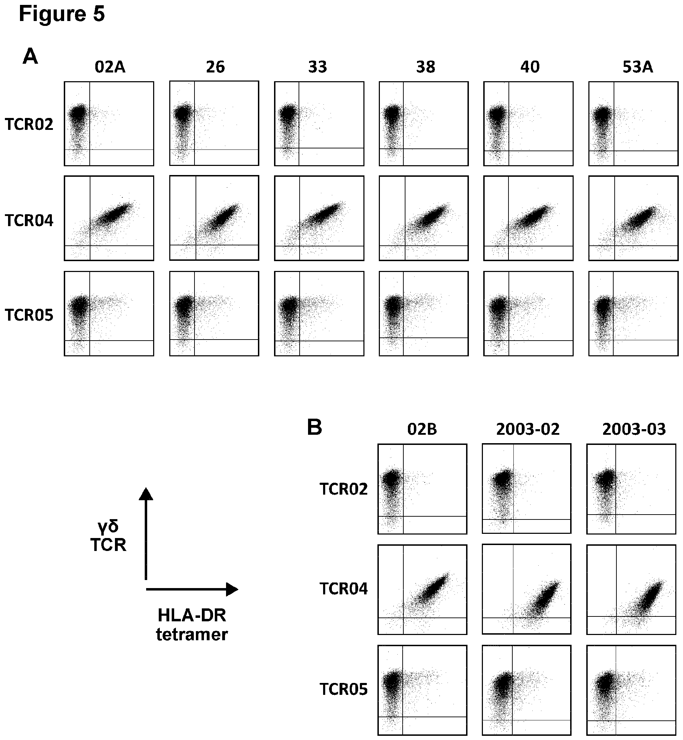

**Fig. 5****: Staining of the JE6.1 cell lines expressing TCR02, TCR04 and TCR05 with HLA-DR tetramers.** The reporter cells were stained with HLA-DR tetramers of the haplotype DRB1*1 (A) or DRB1*3 (B) for two hours. The peptides the tetramers were loaded with are indicated in table 3 (SEQ ID NO: 28-35) as well as their origin.

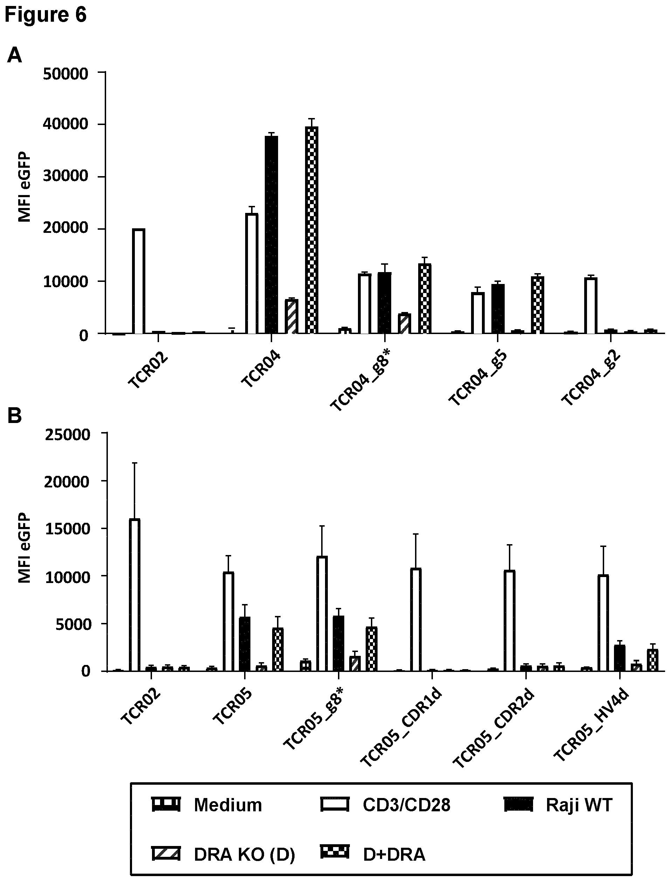

**Fig. 6****: Influence of different part and domains of TCR04 and TCR05 on the recognition of HLA-DR.** A: Different hybrid γδ TCRs as combinations of TCR04 δ-chain with different γ-chains. TCR04_g8* is a Vγ8-chain with the original CDR3γ from the TCR04 Vγ3-chain. B: Hybrid γδ TCRs or sequence chimeras of TCR05 were generated. TCR05_g8* has the same γ-chain as TCR04_g8*. TCR05_CDR1d has its CDR1 in the δ-chain exchanged with the CDR1 from a Vδ3-chain, TCR05_CDR2d has a Vδ2-CDR2 and TCR05_HV4d has a Vδ2-HV4. The Raji cells were co-cultured with the reporter cells at a ratio of 1:1 overnight and the GFP fluorescence as read-out was detected by flow cytometry. MFI: Mean fluorescence intensity.

### Sequences

| | |
|---|---|
| SEQ ID NO: 1 | TRD CDR3 (TCR04) |
| SEQ ID NO: 2 | TRD CDR3 (consensus sequence), X can be any amino acid, preferably, the amino acid in the variable position X is a hydrophobic amino acid, e.g., a non-aromatic hydrophobic amino acid |
| SEQ ID NO: 3 | TRD CDR3 (TCR05) |
| SEQ ID NO: 4 | TRD CDR3 (with ALA) |
| SEQ ID NO: 5 | TRD CDR1 |
| TRD CDR2: | QGS |
| SEQ ID NO: 7 | TRD variable region (TCR04) |
| SEQ ID NO: 8 | TRD variable region na (TCR04) |
| SEQ ID NO: 10 | TRG CDR3 |
| SEQ ID NO: 11 | TRG CDR1 |
| SEQ ID NO: 12 | TRG CDR2 |
| SEQ ID NO: 13 | TRG variable region |
| SEQ ID NO: 14 | TRG variable region nucleic acid |
| SEQ ID NO: 15 | TRD full length (TCR04, aa) |
| SEQ ID NO: 16 | TRG full length (aa) |
| SEQ ID NO: 17 | TRD soluble (TCR04, aa) |
| SEQ ID NO: 18 | TRG soluble (aa) |
| SEQ ID NO: 19 | *Vγ3-forward* primer |
| SEQ ID NO: 20 | *Vγ3-reverse* primer |
| SEQ ID NO: 21 | *Vδ1-forward* primer |
| SEQ ID NO: 22 | *Vδ1-reverse* primer |
| SEQ ID NO: 23 | TRB KO in JE6.1: (gRNA1) |
| SEQ ID NO: 24 | TRB KO in JE6.1: (gRNA2) |
| SEQ ID NO: 25 | HLA-DRA KO in Raji |
| SEQ ID NO: 26 | RFXAP KO in Raji |
| SEQ ID NO: 27 | CDR3 of TCR02 |
| SEQ ID NO: 28-36 | peptides loaded on HLA-DR |

### Examples

### Material and Methods

### Cell lines

The Jurkat reporter cell line JE6.1 NF-κB-eGFP was kindly provided by Peter Steinberger's lab in Vienna, Austria (Rosskopf *et al.,* 2016). The cell lines JE6.1, Raji, K562, U937, THP-1, KG-1, BL64 and CEM were cultivated in RPMI supplemented with 10 % fetal calf serum, 1% penicillin-streptomycin, 2mM L-glutamine and 1 mM sodium pyruvate. HEK293T cells were maintained in DMEM (low-glucose) supplemented with 10% fetal calf serum, 1 % penicillin-streptomycin and 8.75 ml 200 g/l glucose.

### Cloning

The cDNA sequences for the expression of γδ TCRs outside the CDR3 were obtained from the international ImMunoGeneTics information system^{®} (www.imgt.org) and the HLA-DR cDNA sequences were obtained from the Immuno Polymorphism Database (IPD). The CDR3 sequences for the TCR02, TCR04 and TCR05 were described in Ravens et al., 2017. The cDNA sequences were synthesized by BIOCAT and cloned into the retroviral Vectors pBulletIRESneo (γ-chain for TCR and β-chain for HLA-DR) and pBulletIRESpuro (δ-chain for TCR and α-chain for HLA-DR, both vectors kindly provided by Jürgen Kuball's lab, UMC Utrecht, the Netherlands) via the restriction sites for Ncol and BamHI. For the soluble TCR expression, the extracellular domains of the respective γδ TCRs were amplified by PCR by using the following primers:
*Vγ3-forward:* GAAGATCTTCCAGCAATCTGGAGGG (SEQ ID NO: 19)
*Vy3-reverse:* GCTCTAGACTGCAGCAGCAGGGTATC (SEQ ID NO: 20)
*Vδ1-forward:* GAAGATCTGCCCAGAAGGTGACCCAGG (SEQ ID NO: 21)
*Vδ1-reverse:* GCTCTAGACTTCTCTGTGTGCACGATGGC. (SEQ ID NO: 22)

The PCR-product was then cloned into the vector pT1205 via the restriction sites Xbal and BgIII. For the *in silico* design of CDR-exchange mutants, the CDR1 of Vδ1 was replaced by the CDR1 of Vδ3, the CDR2 of Vδ1 by the CDR2 of Vδ2 and the HV4 of Vδ1 by the HV4 of Vδ2. The γ-chain hybrid Vγ8_04* was designed by replacing the CDR3 of a Vγ8-chain with the CDR3 of the Vγ3-chain of the TCRs 02, 04 and 05. The CDR-exchange mutants as well as the hybrid Vγ8_04* were then synthesized by BIOCAT and cloned into the pBullet-vectors respectively.

### Generation of CRISPR-Cas9 Knock out cell lines

For the generation of CRISPR-Cas9 Knock-out cell lines Cas9 ribonucleoparticle preparation and transfection was performed as described elsewhere (Martens *et al.,* 2020). The respective sgRNAs were:
*TRB KO in JE6.1:* GGCTCAAACACAGCGACCTC (gRNA1, SEQ ID NO: 23),
GGCTCTCGGAGAATGACGAG (gRNA2, SEQ ID NO: 24)
*HLA-DRA KO in Raji:* TGCATTGGCCAACATAGCTG (SEQ ID NO: 25)
*RFXAP KO in Raji:* ACACCGCAACAAGATGTACA (SEQ ID NO: 26)

For Jurkat cell nucleofection, the SE cell line nucleofection kit was used, and for Raji cells, the SG cell line nucleofection kit. The cells were cultivated for a week and sorted for negative cells by flow cytometry associated cell sorting and subsequent single cell cloning by limiting dilution. Here, the cells were diluted to reach a density of 5 mL⁻¹. The cell suspension was then distributed over 96-well plates with 100 µL per well and incubated for two weeks. The obtained clones were then screened for the respective knock out by antibody staining. In the case of the Jurkat cells, the aim was to generate an αβ TCR negative cell line for the overexpression of transgenic γδ TCRs. This was achieved by using a crRNA targeting the β-chain locus. The FACS staining was performed with the antibodies: anti-αβ TCR-APC (clone REA652, Miltenyi), anti-CD3-PE-Cy7 (clone: SK7, Miltenyi). The HLA-DR knock out in the Raji cells was generated with crRNAs targeting either the HLA-DR-α chain or the central transcription factor RFXAP. The FACS staining for HLA-DR was performed with the antibody anti-HLA-DRA-APC (clone: REA805, Miltenyi).

### Retroviral transduction

For retroviral particle production 2 x 10⁶ HEK293T cells were seeded in two 10 cm² plate and cultured overnight. The following day, the cells were calcium chloride transfected with the respective pBullet-plasmid, the env-plasmid pMD2.G_VSVg and the gag-pol-plasmid pcDNA3.1MLVwtGag/Pol (both kindly provided by the department of Prof. Dr. Axel Schambach, department of Hematology, Hannover Medical School). The supernatant of the cultures was harvested the next two days and the viral particles were subsequently concentrated by ultracentrifugation at 10000 rpm overnight. The particles were resuspended in RPMI culture medium and stored at -80°C or used immediately. Transduction of JE6.1 and Raji cells was performed by spin infection at 1800 rpm for 2h with 4 nM protamine sulfate and 20 µl of each viral particle suspension to be transduced. Afterwards the cells were cultured for two days with an intermediate medium renewal. To select for successfully transduced cells, 1 ng/µL puromycin and/or 600 ng/µL G418 were added to the culture for one week. Protein expression was determined by antibody staining: γδ TCR expression was detected via anti-γδ TCR-PE (clone: REA591, Miltenyi) and anti-CD3-PE-Cy7 (see above). HLA-DR expression was detected via anti-HLA-DRA-APC (see above).

### Co-culture of JE6.1 reporter cells with target cell lines

JE6.1 reporter cell lines expressing the γδ TCRs 02, 04 and 05 were harvested and brought to a density of 1 x 10⁶ mL⁻¹ with RPMI culture medium without antibiotics. The respective target cell lines Raji and derivatives, K562, U937, BL64, KG-1, THP-1 and CEM were harvested and prestained with the cell proliferation dye eFluor 670 (Invitrogen) according to the manufacturer's protocol to allow for distinction of target versus reporter cells in flow cytometry. Afterwards, the cell lines were brought to a density of 1 x 10⁶ mL⁻¹. Target and reporter cells were co-cultured at a ratio of 1:1 with 5 x 10⁴ cells on both sides. The co-culture volume was 100 µL in a 96-well plate. The negative control were reporter cells without target cells and the positive control were beads coated with anti-CD3/anti-CD28 antibodies (Treg Expansion Kit, Miltenyi) for the stimulation of TCR signaling. The cells were co-cultured overnight and the GFP fluorescence was detected the next day via flow cytometry.

### Soluble TCR tetramer production

The vectors for the expression of soluble TCR versions of TCR02 and TCR04 were transfected into Schneider cells *(Drosophila melanogaster).* The expression of the sTCRs was induced by addition of 500 µM CuSO₄, and the soluble proteins were sequestered into the supernatant. Affinity purification of the sTCRs was performed via their C-terminal Twin-Strep-tag. After a subsequent size exclusion chromatography the sTCRs were concentrated and stored in 10 mM Tris pH 8.0, 100 mM NaCl at -80°C. For tetramerization with streptavidin-PE (Invitrogen), the ratio of sTCR:streptavidin-PE was 4:1. 4.07 µg sTCR were added to 4 µL streptavidin-PE and incubated for 45 minutes on ice. Afterwards 1.325 µL of the tetramer suspension could be used to stain up to 1 x 10⁶ cells per sample in PBS + 1 % fetal calf serum (FCS). After 20 minutes on ice, the cells were washed once with PBS + 1 % FCS and the fluorescence detected by flow cytometry.

### Genome-wide CRISPR-Cas9 Knock-out Screening

In order to identify the antigen for TCR04 and TCR05, a Genome-wide Knock out Screening with the Raji cell line was performed. The Human Brunello CRISPR knockout pooled library was a gift from David Root and John Doench (Addgene #73178). The transduction of the library was performed as described elsewhere (Doench *et al*., 2016). Half of every sample were directly submitted to genomic DNA isolation and the other half was stained with sTCR tetramers as stated above. By fluorescence associated cell sorting the lowest 10 % of the stained cells and cultured for one week in RPMI culture medium. The staining and sorting procedures were repeated three more times to select for sTCR negative cells. Genomic DNA was isolated from the sorted cells and the sgRNA sequences were PCR-amplified according to the protocol provided with the sgRNA library by David Root and John Doench (cf. Doench et al., 2016 or Addgene: Broad GPP - Human Genome-wide CRISPR Knockout Libraries). In brief, the sgRNA encoding sequences were amplified with Primer-pairs containing the respective Illumina sequencing adapters. The PCR products were purified with AMPure XP magnetic beads and the DNA concentration for sequencing was determined with Quant-iT^{™} Picogreen^{™} and a Qubit 4 fluorometer (Thermo Fisher). The PCR library was subsequently sequenced by Illumina NovaSeq and the bioinformatic analysis for the identification of enriched sgRNAs was performed with the edgeR package in R.

### Results

To identify novel antigens from different γδ TCRs, we expressed the sequences of pairs of one γ-and one δ-chain each in a human T-cell line (Jurkat, JE6.1). A reporter gene construct of GFP under the control of an NF-κB-specific promoter is included in this cell line (Rosskopf et al., 2016), allowing detection of activation by the corresponding expressed TCR. When the γδ TCR is activated, the cells express GFP, which in turn can be measured by flow cytometry. The generated cell lines were co-cultured with different tumor cell lines to detect specific reactivities against them. Here, we found a Vγ3Vδ1⁺ γδ TCR (TCR04) that can specifically recognize B cell lines (Raji, BL64) (Figure 1). Especially the CDR3 region of the δ-chain plays a crucial role, since another γδ TCR (TCR02), which is identical to TCR04 except for the CDR3δ, does not recognize the mentioned cell lines (Table 1). Furthermore, another Vγ3Vδ1⁺ TCR (TCR05) which differs in the amino acid 118 (as counted from the starting methionine) from TCR04 is equally reactive to B cell lines despite with a lower magnitude.

**Table 1: CDR3 sequences of γ- and 5-chain of TCR02, TCR04 and TCR05.**

| **CDR3γ** | **CDR3δ** |
|---|---|
| **All TCRs**: ATWDRGRKLF (SEQ ID NO: 10) | **TCR02:** ALGELPRHHLTDKLIF (SEQ ID NO: 27) |
| | **TCR04:** ALGELYVPLYWGPTPRTTDKLIF (SEQ ID NO: 1) |
| | **TCR05:** ALGELYGPLYWGPTPRTTDKLIF (SEQ ID NO: 3) |

For ligand identification approaches we chose to continue with the highly reactive TCR04 because the co-culture results suggested a similar target specificity for both TCR04 and TCR05. In order to characterize the binding of TCR04 to cell lines and identify the antigen, we expressed the extracellular domain of the γδ TCR as a soluble protein in insect cells. Via a C-terminal Strep tag, the soluble TCR can be bound to fluorophore-coupled streptavidin. This leads to staining of the cells upon specific γδ TCR binding. Here, the soluble form of TCR04 was also shown to bind specifically to the Raji B cell line (Figure 2).

Streptavidin has four binding sites for Strep tags, and incubation with the soluble TCR thus produces tetramers. This increases the avidity of the polyvalent TCR complex to the target structures, which is necessary for many known αβ- and γδ-chains, as the affinity of antigen binding would be too low for detection by flow cytometry. However, the binding of the γδ TCR04 to the Raji B cell line has sufficient affinity to bind these tumor cells as both a monomer and a tetramer (Figure 2). For this reason, we hypothesize that the binding affinity of TCR04 is similar to that of an antibody and thus significantly higher than that of most known TCRs.

To identify the specific antigen recognized by TCR04, we made use of a genome-wide CRISPR Cas9 Knock-out screen. A library of about 76000 different single gRNAs (sgRNAs) was used to transduce the Raji B cell line targeting roughly 19000 genes of the human genome. By selecting those cells which lost the binding of TCR04, we enriched for cells containing the causative sgRNAs (Figure 3A). Subsequently, the respective sgRNAs were identified by next generation sequencing (Table 2).

**Table 2: Genes enriched in Genome-wide CRISPR-Cas9 Knock out Screening.**

| | **NGenes** | **Direction** | **PValue** | **FDR** |
|---|---|---|---|---|
| **CIITA** | 4 | Up | 9.01 E-24 | 4.60E-20 |
| **HLA-DRA** | 4 | Up | 8.51 E-19 | 2.18E-15 |
| **RFXAP** | 4 | Up | 3.58E-16 | 6.10E-13 |
| **RFX5** | 4 | Up | 6.27E-15 | 8.02E-12 |
| **RFXANK** | 4 | Up | 3.84E-13 | 3.93E-10 |
| **ARID1A** | 4 | Up | 1.22E-09 | 1.04E-06 |
| **GCSAM** | 4 | Up | 1.36E-07 | 9.92E-05 |
| **DACT3** | 4 | Up | 2.35E-07 | 0.00015018 |
| **SPI1** | 4 | Up | 2.77E-06 | 0.00157391 |
| **TAF6L** | 4 | Up | 3.57E-06 | 0.00182437 |

Using this method, we identified HLA-DR and its associated transcriptional regulators as genes critical for binding of the soluble γδ TCR. To verify these results, we again knocked out HLA-DR separately by targeting the HLA-DR-α chain with CRISPR-Cas9 specifically and tested the reactivity of both TCR04 and TCR05 (Figure 3B). When HLA-DR was absent, the reactivity was either lost for TCR05 or significantly reduced for TCR04 (see Figure 3C). Furthermore, the reintroduction of the gene for the HLA-DR-α chain rescued the specific reactivity of both TCR04 and TCR05.

HLA-DR as other MHC molecules comprises a high degree of combinatorial diversity of an invariant α-chain with a diverse β-chain. As every individual expresses its own specific subset of MHC molecules, it is of interest for a putative application of these γδ TCRs in immunotherapy as to whether the MHC-haplotype impacts the reactivities of TCR04 and TCR05. Moreover, a possible role of peptides presented by HLA-DR has to be considered. To address the influence of HLA-DR haplotype and presented peptide on the TCRs' reactivities, we employed HLA-DR-tetramer staining and transgenic overexpression of different HLA-DR haplotypes in an HLA-DR deficient cell line. The loss of endogenous α- and β-chain expression were achieved by knocking out RFXAP, a central transcription factor for MHC class II expression, in Raji cells. Thus, upon retroviral transduction, the cells only express the introduced transgenic HLA-DR and the results can therefore be attributed to the respective haplotype tested. Raji cells endogenously express HLA-DRB1*3, HLA-DRB1*10 and HLA-DRB3*2, which is why they were included in this screening. Furthermore, we included HLA-DRB1*1 and HLA-DRB1*4, as they were not supposed to be initially present in Raji cells, but sequence-wise similar to the other HLA-DR versions. The subsequent co-culture with the reporter cell lines revealed that all five HLA-DR isoforms were able to stimulate TCR04 and rescue the knock out phenotype (Figure 4). Hence, we considered its reactivity as independent from the HLA-DR haplotype. The HLA-DR recognition by TCR05 was impaired with all different HLA-DR isoforms tested (Figure 4). Possibly, this TCR requires functional peptide loading of HLA-DR for its activation. The transcription factor RFXAP, which has been knocked out in this cell line, regulates not only the expression of MHC class II but also of its peptide loading machinery. Consequently, functional peptide loading remains impaired despite the expression of transgenic HLA-DR, and this in return interferes with antigen recognition by TCR05. Whether however a specific peptide is required or rather an array of peptides with certain physico-chemical constraints are preferred remains unclear so far.

As a second approach to further test the influence of the peptide presented by HLA-DR on the recognition by TCR04 and TCR05, we employed staining with HLA-DR tetramers loaded with different peptides. The HLA-DR isoforms in these tetramers were either HLA-DRB1*1 or HLA-DRB1*3 and they contained common antigenic peptides from CMV, EBV, house dust mite, melanoma and the CLIP peptide (see Table 3).

**Table 3: Haplotype and bound peptide of the HLA-DR tetramers used to stain JE6. 1 cells expressing the investigated yδ TCRs.**

| **Haplotype** | **#** | **Peptide (SEQ ID NO)** | **Peptide origin** |
|---|---|---|---|
| DRB1*01:01 | 02A | PVSKMRMATPLLMQ (28) | CLIP 87-101 |
| DRB1*03:01 | 02B | PVSKMRMATPLLMQA (29) | CLIP 87-101 |
| DRB1*01:01 | 26 | LPLKMLNIPSINVH (30) | CMV pp65 116-129 |
| DRB1*01:01 | 33 | ACYEFLWGPRALVETS (31) | MAGE-A3 267-282 |
| DRB1*01:01 | 38 | TSLYNLRRGTALA (32) | EBV EBNA1 515-527 |
| DRB1*01:01 | 40 | TVFYNIPPMPL (33) | EBV EBNA2 280-290 |
| DRB1*01:01 | 53A | LRQMRTVTPIRMQGG (34) | House dust mite Der p1 96-110 |
| DRB1*03:01 | 2003-02 | TRRGRVKIDEVSRMF (35) | HCMV IE2 |
| DRB1*03:01 | 2003-03 | VKQIKVRVDMVRHRI (36) | HCMV IE1 |

The staining of the reporter cells expressing TCR02, TCR04 and TCR05 with the respective tetramers revealed that TCR04 binds all HLA-DR isoforms irrespective of the peptide presented, and we therefore consider its HLA-DR reactivity haplotype- and peptide-independent (Figure 5A and 5B). TCR05 did not bind any of the peptide, presumably because of its low affinity towards HLA-DR that did not allow for a detection of the interaction by flow cytometry.

Although the comparison of TCR04 and TCR05 with the non-reactive yet very similar TCR02 suggested a major relevance of the δ-chain CDR3, a function of other CDRs and the γ-chain cannot be excluded. We therefore aimed at investigating the role of other domains of the γδ TCRs by generating CDR exchange mutants or chain swapping experiments. To test for redundancy of the γ-chain, which is identical between TCR02, TCR04 and TCR05, we paired the δ-chain of TCR04 with chains of Vγ2, Vγ5 and Vγ8 derived from other γδ TCR combinations. The Vγ5-chain (TCR04_g5) was sequence- and length-wise similar to the original Vγ3-chain whereas the Vγ2-chain (TCR04_g2) was not. To avoid effects merely based on different CDR3 lengths, a combination of the γ-chain CDR3 from TCR04 inside a Vγ8-chain was designed as a fourth option (TCR04_g8*). For TCR05, the γ-chain swapping was performed only for TCR04_g8*. In the co-culture with Raji cells, the TCR04 δ-chain combinations TCR04_g8* and TCR04_g5 retained their reactivity to HLA-DR despite with a lower magnitude (Figure 6A). The same was true for TCR05 in combination with TCR05_g8* (Figure 6B). Together with Vγ2, the reactivity of TCR04 was nearly completely lost. This indicates that the γ-chain of TCR04 is not completely redundant. Whether this is due to specific sequence requirements or rather the CDR3 length cannot be deduced from the experiments performed so far.

To further examine the sequence requirements in the δ-chain, the CDR1, CDR2 and the hypervariable region 4 (HV4) of TCR05 were exchanged for respective sequences from other Vδ-fragments. The exchange of HV4 lowered the reactivity but did not abolish it indicating that the HV4 is not directly implicated in the interaction between TCR05 and HLA-DR (Figure 6B). The reduced reactivity might also be explained by structural differences due to the exchange. With the CDR1 and CDR2 being exchanged however, the recognition of HLA-DR by TCR05 was completely lost. We therefore concluded that, although the δ-chain CDR3 is a major prerequisite for the activation of TCR05 by HLA-DR, CDR1 and CDR2 of the Vδ1-chain are equally required for the proper interaction.

### Conclusion

The Vγ3Vδ1⁺ TCRs TCR04 and TCR05 recognize HLA-DR as a target on B cell lines. The difference at position 118 from a Valine in TCR04 to a Glycine in TCR05 leads to an increased affinity of TCR04 towards HLA-DR. The Knock-out of HLA-DR-α ablates or reduces the reactivities of both TCRs whereas a re-expression restores recognition. The activation of TCR04 is furthermore independent of the HLA-DR subtype and the presented peptide, whereas TCR05 depends on a functional peptide processing within the target cell.

Besides the δ-chain CDR3, the TCR05 requires the Vδ1 CDR1 and CDR2 for a cognate interaction with HLA-DR. Whether this can be extended to TCR04 has not been tested yet. The Vγ3-chain plays a less important role for the recognition of HLA-DR, but is not completely redundant. Especially the CDR3 length and possibly also the sequence are relevant for the interaction.

The HLA-DR specificity of TCR04 has a high therapeutic potential, because the binding is independent of the HLA-DR haplotype and thus would occur in the same way in each individual. Equally, TCR05 might be used in a more specific, narrow application with certain HLA-DR haplotypes and presented peptides. In addition, the high affinity of the binding of TCR04 strongly resembles that of CARs, which have been successfully used in anti-tumor therapy. TCR04, as a transgenic receptor on T or NK cells could also recognize specific HLA-DR-positive target cells and trigger a cytotoxic and inflammatory immune response that ultimately leads to tumor cell destruction. In addition to its use in targeting HLA-DR-positive tumor diseases such as myeloid and B-cell leukemias, this receptor TCR04 may also find application in the therapy of other inflammatory diseases caused by B cells or macrophages. In addition to the expression of the γδ TCR04, e.g., in T cells, its high affinity could also allow it to be used in soluble form for therapeutic purposes, such as part of a bi-specific antibody to eliminate HLA-DR expressing tumor cells, but also immunomodulatory to regulate excessive HLA-DR-dependent immune responses.

### References

Brudno, J. N. et al. (2020) 'Safety and feasibility of anti-CD19 CAR T cells with fully human binding domains in patients with B-cell lymphoma', Nature Medicine. Springer US, 26(2), pp. 270-280. doi: 10.1038/s41591-019-0737-3.
de Bruin, R. C. G. et al. (2018) 'A bispecific nanobody approach to leverage the potent and widely applicable tumor cytolytic capacity of Vγ9Vδ2-T cells', Oncolmmunology. Taylor & Francis, 7(1), pp. 1-14. doi: 10.1080/2162402X.2017.1375641.
Deseke, M. and Prinz, I. (2020) 'Ligand recognition by the γδ TCR and discrimination between homeostasis and stress conditions', Cellular and Molecular Immunology. Springer US, 17(9), pp. 914-924. doi: 10.1038/s41423-020-0503-y.
Doench, J. G. et al. (2016) 'Optimized sgRNA design to maximize activity and minimize off-target effects of CRISPR-Cas9', Nature Biotechnology. Nature Publishing Group, 34(2), pp. 184-191. doi: 10.1038/nbt.3437.
Doran, S. L. et al. (2019) 'T-Cell Receptor Gene Therapy for Human Papillomavirus-Associated Epithelial Cancers: A First-in-Human, Phase I/II Study', Journal of Clinical Oncology, 37(30), pp. 2759-2768. doi: 10.1200/JCO.18.02424.
Engels, B. et al. (2003) 'Retroviral Vectors for High-Level Transgene Expression in T Lymphocytes', Human Gene Therapy, 14(12), pp. 1155-1168. doi: 10.1089/104303403322167993.
Flament, C. et al. (1994) 'Human TCR-γ/δ alloreactive response to HLA-DR molecules: Comparison with response of TCR-α/β', The Journal of Immunology, 153(7), pp. 2890-2904.
Leisegang, M. et al. (2008) 'Enhanced functionality of T cell receptor-redirected T cells is defined by the transgene cassette', Journal of Molecular Medicine, 86(5), pp. 573-583. doi: 10.1007/s00109-008-0317-3.
Martens, R. et al. (2020) 'Efficient homing of T cells via afferent lymphatics requires mechanical arrest and integrin-supported chemokine guidance', Nature Communications, 11(1). doi: 10.1038/s41467-020-14921-w.
Mensali, N. et al. (2019) 'NK cells specifically TCR-dressed to kill cancer cells', EBioMedicine, 40, pp. 106-117. doi: 10.1016/j.ebiom.2019.01.031.
Oberg, H. H. et al. (2014) 'Novel bispecific antibodies increase gd t-cell cytotoxicity against pancreatic cancer cells', Cancer Research, 74(5), pp. 1349-1360. doi: 10.1158/0008-5472.CAN-13-0675.
Oberg, H. H. et al. (2015) 'γδ T cell activation by bispecific antibodies', Cellular Immunology, 296(1), pp. 41-49. doi: 10.1016/j.cellimm.2015.04.009.
Oberg, H. H. et al. (2020) 'Bispecific antibodies enhance tumor-infiltrating T cell cytotoxicity against autologous HER-2-expressing high-grade ovarian tumors', Journal of Leukocyte Biology, 107(6), pp. 1081-1095. doi: 10.1002/JLB.5MA1119-265R.
Ravens, S. et al. (2017) 'Human γδ T cells are quickly reconstituted after stem-cell transplantation and show adaptive clonal expansion in response to viral infection', Nature Immunology, 18(4), pp. 393-401. doi: 10.1038/ni.3686.
Robinson, R. A. et al. (2021) Engineering soluble T-cell receptors for therapy', The FEBS Journal, p. febs.15780. doi: 10.1111/febs.15780.
Rosskopf, S. et al. (2016) Creation of an engineered APC system to explore and optimize the presentation of immunodominant peptides of major allergens', Scientific Reports. Nature Publishing Group, 6(1), p. 31580. doi: 10.1038/srep31580.
Scholten, K. B. J. et al. (2006) 'Codon modification of T cell receptors allows enhanced functional expression in transgenic human T cells', Clinical Immunology, 119(2), pp. 135-145. doi: 10.1016/j.clim.2005.12.009.
Sebestyen, Z. et al. (2020) 'Translating gammadelta (γδ) T cells and their receptors into cancer cell therapies', Nature Reviews Drug Discovery, 19(3), pp. 169-184. doi: 10.1038/s41573-019-0038-z.
WO 2017212072 A1
US 20200316124 A1

## Claims

1. A pharmaceutical composition comprising
a) a nucleic acid encoding a TCR gamma chain construct (TRG) and a TCR delta chain construct (TRD) of a TCR construct;
b) a TCR construct comprising a TRG and a TRD; and/or
c) a human lymphoid host cell comprising the nucleic acid(s) of a) and expressing the TCR construct of b);
wherein the TCR construct is specific for HLA-DR and wherein the TRD comprises a CDR3 having at least 95% sequence identity to SEQ ID NO: 1, a CDR1 having at least 80% sequence identity to SEQ ID NO: 5 and a CDR2 having at least 66% sequence identity to QGS,
wherein, preferably, the TRD comprises a CDR3 having SEQ ID NO: 2.

2. A TCR construct comprising a TRG and a TRD, wherein the TCR construct is specific for HLA-DR and wherein the TRD comprises a CDR3 having at least 95% sequence identity to SEQ ID NO: 1, a CDR1 having at least 80% sequence identity to SEQ ID NO: 5 and a CDR2 having at least 66% sequence identity to QGS, and wherein the TCR construct is
a) a soluble TCR construct and/or
b) a bispecific TCR construct and/or
c) a chimeric TCR construct and/or
d) a scTCR construct.

3. An expression vector encoding a TCR gamma chain construct (TRG) and a TCR delta chain construct (TRD) of a TCR construct specific for HLA-DR, wherein the TRD comprises a CDR3 having at least 95% sequence identity to SEQ ID NO: 1, a CDR1 having at least 80% sequence identity to SEQ ID NO: 5 and a CDR2 having at least 66% sequence identity to QGS,
wherein expression or the TRG and/or TRD is controlled by a heterologous promotor capable of mediating expression in a lymphoid cell, and/or wherein the TCR construct is the TCR construct of claim 2,
wherein, preferably, expression or the TRG and/or TRD is controlled by a heterologous promotor.

4. A host cell comprising the expression vector of claim 3, wherein the host cell preferably is a human lymphoid host cell.

5. The pharmaceutical composition of claim 1, comprising the TCR construct of claim 2, the expression vector of claim 3, or the host cell of claim 4.

6. The pharmaceutical composition of any of claims 1 or 5, the TCR construct of any of claims 2 or 5, the expression vector of any of claims 3 or 5, the host cell of any of claims 4-5,
wherein the TRD comprises a CDR3 having SEQ ID NO: 2, wherein, optionally, the amino acid in the variable position X is a hydrophobic amino acid, preferably, a non-aromatic hydrophobic amino acid such as Val, Ala, Gly, Ile, Leu or Val.

7. The pharmaceutical composition of any of claims 1 or 5-6, the TCR construct of any of claims 2 or 5-6, the expression vector of any of claims 3 or 5-6, the host cell of any of claims 4-6, wherein the TRD comprises a CDR3 having SEQ ID NO: 1.

8. The pharmaceutical composition of any of claims 1 or 5-6, the TCR construct of any of claims 2 or 5-6, the expression vector of any of claims 3 or 5-6, the host cell of any of claims 4-6, wherein the TRD comprises a CDR3 having SEQ ID NO: 3 or 4, optionally, SEQ ID NO: 3.

9. The pharmaceutical composition of any of claims 1 or 5-8, the TCR construct of any of claims 2 or 5-8, the expression vector of any of claims 3 or 5-8, the host cell of any of claims 4-8, wherein the TRD is a Vδ1D2J1 TRD.

10. The pharmaceutical composition of any of claims 1 or 5-9, the TCR construct of any of claims 2 or 5-9, the expression vector of any of claims 3 or 5-9, the host cell of any of claims 4-9, wherein the TRD comprises a variable region having at least 80% sequence identity to SEQ ID NO: 7, wherein the TRD is optionally encoded by a nucleic acid having at least 80% sequence ideintity to SEQ ID NO: 8.

11. The pharmaceutical composition of any of claims 1 or 5-10, the TCR construct of any of claims 2 or 5-10, the expression vector of any of claims 3 or 5-10, the host cell of any of claims 4-10, wherein the TRG is selected from the group consisting of a Vγ3 TRG, a Vγ5 TRG or a Vγ8 TRG, preferably, a Vy3J1 TRG.

12. The pharmaceutical composition of any of claims 1 or 5-11, the TCR construct of any of claims 2 or 5-11, the expression vector of any of claims 3 or 5-11, the host cell of any of claims 4-11, wherein the TRG and/or the TRD further comprise a constant region selected from the group comprising a human constant region, a murine constant region or a chimeric constant region.

13. The pharmaceutical composition of any of claims 1 or 5-12, the TCR construct of any of claims 2 or 5-12, the expression vector of any of claims 3 or 5-12, or the host cell of any of claims 4-12, wherein the TRG and the TRD form a soluble heterodimer, optionally, associated with a further agent selected from the group comprising a fluorescent label, a tag, and a lipidic moiety.

14. The pharmaceutical composition of any of claims 1 or 5-13, the expression vector of any of claims 3 or 5-13, or the host cell of any of claims 4-13, wherein the nucleic acid is selected from the group consting of a viral vector, a transposon, a vector suitable for CRISPR/CAS based recombination or a plasmid suitable for *in vitro* RNA transcription, or wherein the nucleic acid is DNA integrated into the genomic DNA of a host cell.

15. The pharmaceutical composition of any of claims 1 or 5-14, the expression vector of any of claims 3 or 5-14, or the host cell of any of claims 4-12, wherein the host cell is a human T cell, NK cell or NK/T cell.

16. The pharmaceutical composition of any of claims 1 or 5-15 for use in treatment of a human patient having a lymphoproliferatve disorder associated with abnormal proliferation of HLA-DR+ cells,
preferably, a malignancy of the hematopoietic and lymphoid tissues selected from the group comprising lymphoma, e.g., Hodgkin's lymphoma or Non-Hodgkin's lymphoma, leukemia, e.g., acute lymphoblastic leukemia (ALL), acute myelogenous leukemia (AML), chronic lymphocytic leukemia (CLL), chronic myelogenous leukemia (CML), or acute monocytic leukemia (AMoL), myeloma, e.g., multiple myeloma, and follicular dendritic cell sarcoma,

17. The pharmaceutical composition for use of claim 16, wherein the treatment is an immunotherapy selected from the group comprising adoptive T cell therapy, TCR gene therapy, or a treatment involving targeting of HLA-DR+ cells with the TCR construct in soluble form or as a bispecific antibody, preferably, adoptive T cell therapy.

18. A method of detecting or targeting a HLA-DR+ cell, comprising contacting said cell with the TCR construct of any of claims 2 or 5-13, or the host cell of any of claims 4-15, preferably, said TCR construct.
wherein said method optionally is an *in vitro* method comprising contacting a sample from a human with said TCR construct or said host cell.


SEQUENCE LISTING

<110> Medizinische Hochschule Hannover

<120> HLA-DR-specific gamma delta TCR construct and use thereof

<130> 13210

<160> 36

<170> BiSSAP 1.3.6

<210> 1
<211> 23
<212> PRT
<213> Homo sapiens

<220>
<223> TRD CDR3 (TCR04)

<400> 1
Ala Leu Gly Glu Leu Tyr Val Pro Leu Tyr Trp Gly Pro Thr Pro Arg
1               5                   10                  15
Thr Thr Asp Lys Leu Ile Phe
            20

<210> 2
<211> 23
<212> PRT
<213> Homo sapiens

<220>
<223> TRD CDR3 (consensus sequence)

<220>
<221> VARIANT
<222> 7
<223> X can be any amino acid, preferably, the amino acid in the
      variable position X is a hydrophobic amino acid, e.g., a
      non-aromatic hydrophobic amino acid (i.e., Val, Ala, Gly, Ile,
      Leu, Val or Met)

<400> 2
Ala Leu Gly Glu Leu Tyr Xaa Pro Leu Tyr Trp Gly Pro Thr Pro Arg
1               5                   10                  15
Thr Thr Asp Lys Leu Ile Phe
            20

<210> 3
<211> 23
<212> PRT
<213> Homo sapiens

<220>
<223> TRD CDR3 (TCR05)

<400> 3
Ala Leu Gly Glu Leu Tyr Gly Pro Leu Tyr Trp Gly Pro Thr Pro Arg
1               5                   10                  15
Thr Thr Asp Lys Leu Ile Phe

            20

<210> 4
<211> 23
<212> PRT
<213> Homo sapiens


<220>
<223> TRD CDR3 (with ALA)

<400> 4
Ala Leu Gly Glu Leu Tyr Ala Pro Leu Tyr Trp Gly Pro Thr Pro Arg
1               5                   10                  15
Thr Thr Asp Lys Leu Ile Phe
            20

<210> 5
<211> 7
<212> PRT
<213> Homo sapiens


<220>
<223> TRD CDR1

<400> 5
Thr Ser Trp Trp Ser Tyr Tyr
1               5

<210> 6
<400> 6
000

<210> 7
<211> 94
<212> PRT
<213> Homo sapiens


<220>
<223> TRD variable region (TCR04)

<400> 7
Ala Gln Lys Val Thr Gln Ala Gln Ser Ser Val Ser Met Pro Val Arg
1               5                   10                  15
Lys Ala Val Thr Leu Asn Cys Leu Tyr Glu Thr Ser Trp Trp Ser Tyr
            20                  25                  30
Tyr Ile Phe Trp Tyr Lys Gln Leu Pro Ser Lys Glu Met Ile Phe Leu
        35                  40                  45
Ile Arg Gln Gly Ser Asp Glu Gln Asn Ala Lys Ser Gly Arg Tyr Ser
    50                  55                  60
Val Asn Phe Lys Lys Ala Ala Lys Ser Val Ala Leu Thr Ile Ser Ala
65                  70                  75                  80
Leu Gln Leu Glu Asp Ser Ala Lys Tyr Phe Cys Ala Leu Gly
                85                  90

<210> 8
<211> 282
<212> DNA
<213> Homo sapiens

<220>
<223> TRD variable region na (TCR04)

<400> 8
gcccagaagg tgacccaggc ccagtcctcc gtgtccatgc ctgtgagaaa ggccgtgacc      60

ctgaattgtc tgtacgagac aagctggtgg tcctactaca tcttttggta caagcagctg     120

ccttccaagg agatgatctt tctgatcaga cagggcagcg atgagcagaa cgccaagtcc     180

ggcaggtact ccgtgaactt taagaaggcc gccaagtccg tggccctgac aatcagcgcc     240

ctgcagctgg aggatagcgc caagtacttt tgcgccctgg gc                        282


<210> 9
<211> 10
<212> PRT
<213> Homo sapiens


<220>
<223> TRG CDR3

<400> 9
Ala Thr Trp Asp Arg Gly Arg Lys Leu Phe
1               5                   10

<210> 10
<211> 10
<212> PRT
<213> Homo sapiens


<220>
<223> TRG CDR3

<400> 10
Ala Thr Trp Asp Arg Gly Arg Lys Leu Phe
1               5                   10

<210> 11
<211> 6
<212> PRT
<213> Homo sapiens


<220>
<223> TRG CDR1

<400> 11
Val Thr Asn Thr Phe Tyr
1               5

<210> 12
<211> 8
<212> PRT
<213> Homo sapiens


<220>
<223> TRG CDR2

<400> 12
Tyr Asp Val Ser Thr Ala Arg Asp
1               5

<210> 13
<211> 100
<212> PRT
<213> Homo sapiens

<220>
<223> TRG variable region

<400> 13
Ser Ser Asn Leu Glu Gly Arg Thr Lys Ser Val Thr Arg Gln Thr Gly
1               5                   10                  15
Ser Ser Ala Glu Ile Thr Cys Asp Leu Thr Val Thr Asn Thr Phe Tyr
            20                  25                  30
Ile His Trp Tyr Leu His Gln Glu Gly Lys Ala Pro Gln Arg Leu Leu
        35                  40                  45
Tyr Tyr Asp Val Ser Thr Ala Arg Asp Val Leu Glu Ser Gly Leu Ser
    50                  55                  60
Pro Gly Lys Tyr Tyr Thr His Thr Pro Arg Arg Trp Ser Trp Ile Leu
65                  70                  75                  80
Arg Leu Gln Asn Leu Ile Glu Asn Asp Ser Gly Val Tyr Tyr Cys Ala
                85                  90                  95
Thr Trp Asp Arg
            100

<210> 14
<211> 300
<212> DNA
<213> Homo sapiens

<220>
<223> TRG variable region nucleic acid

<400> 14
tccagcaatc tggagggcag gacaaagtcc gtgacaaggc agacaggctc cagcgccgag      60

atcacatgcg acctgacagt gacaaatacc ttctacatcc actggtacct gcaccaggag     120

ggcaaggccc ctcagagact gctgtactac gacgtgagca cagccaggga cgtgctggag     180

tccggcctgt cccctggcaa gtactacaca cacaccccta gaagatggag ctggatcttg     240

agactgcaga atctgatcga gaacgattcc ggcgtgtact actgcgccac atgggataga     300

<210> 15
<211> 305
<212> PRT
<213> Homo sapiens

<220>
<223> TRD full length (TCR04, aa)

<400> 15
Met Gly Trp Ala Leu Val Val Leu Leu Ala Phe Leu Ser Pro Ala Ser
1               5                   10                  15
Gln Lys Ser Ser Asn Leu Glu Gly Arg Thr Lys Ser Val Thr Arg Gln

20                          25                          30
Thr Gly Ser Ser Ala Glu Ile Thr Cys Asp Leu Thr Val Thr Asn Thr
        35                          40                          45
Phe Tyr Ile His Trp Tyr Leu His Gln Glu Gly Lys Ala Pro Gln Arg
    50                          55                          60
Leu Leu Tyr Tyr Asp Val Ser Thr Ala Arg Asp Val Leu Glu Ser Gly
65                          70                          75                          80
Leu Ser Pro Gly Lys Tyr Tyr Thr His Thr Pro Arg Arg Trp Ser Trp
                        85                          90                          95
Ile Leu Arg Leu Gln Asn Leu Ile Glu Asn Asp Ser Gly Val Tyr Tyr
                100                         105                         110
Cys Ala Thr Trp Asp Arg Gly Arg Lys Leu Phe Gly Ser Gly Thr Thr
        115                         120                         125
Leu Val Val Thr Asp Lys Gln Leu Asp Ala Asp Val Ser Pro Lys Pro
    130                         135                         140
Thr Ile Phe Leu Pro Ser Ile Ala Glu Thr Lys Leu Gln Lys Ala Gly
145                         150                         155                         160
Thr Tyr Leu Cys Leu Leu Glu Lys Phe Phe Pro Asp Val Ile Lys Ile
                        165                         170                         175
His Trp Gln Glu Lys Lys Ser Asn Thr Ile Leu Gly Ser Gln Glu Gly
                180                         185                         190
Asn Thr Met Lys Thr Asn Asp Thr Tyr Met Lys Phe Ser Trp Leu Thr
        195                         200                         205
Val Pro Glu Lys Ser Leu Asp Lys Glu His Arg Cys Ile Val Arg His
    210                         215                         220
Glu Asn Asn Lys Asn Gly Val Asp Gln Glu Ile Ile Phe Pro Pro Ile
225                         230                         235                         240
Lys Thr Asp Val Ile Thr Met Asp Pro Lys Asp Asn Cys Ser Lys Asp
                        245                         250                         255
Ala Asn Asp Thr Leu Leu Leu Gln Leu Thr Asn Thr Ser Ala Tyr Tyr
                260                         265                         270
Met Tyr Leu Leu Leu Leu Leu Lys Ser Val Val Tyr Phe Ala Ile Ile
        275                         280                         285
Thr Cys Cys Leu Leu Arg Arg Thr Ala Phe Cys Cys Asn Gly Glu Lys
    290                         295                         300
Ser
305

<210> 16
<211> 298
<212> PRT
<213> Homo sapiens


<220>
<223> TRG full length (aa)

<400> 16
Met Val Phe Ser Ser Leu Leu Cys Val Phe Val Ala Phe Ser Tyr Ser
1                   5                   10                  15
Gly Ser Ser Val Ala Gln Lys Val Thr Gln Ala Gln Ser Ser Val Ser
                20                          25                          30
Met Pro Val Arg Lys Ala Val Thr Leu Asn Cys Leu Tyr Glu Thr Ser
        35                          40                          45
Trp Trp Ser Tyr Tyr Ile Phe Trp Tyr Lys Gln Leu Pro Ser Lys Glu
    50                          55                          60
Met Ile Phe Leu Ile Arg Gln Gly Ser Asp Glu Gln Asn Ala Lys Ser
65                          70                          75                          80
Gly Arg Tyr Ser Val Asn Phe Lys Lys Ala Ala Lys Ser Val Ala Leu
                        85                          90                          95
Thr Ile Ser Ala Leu Gln Leu Glu Asp Ser Ala Lys Tyr Phe Cys Ala
                100                         105                         110
Leu Gly Glu Leu Tyr Val Pro Leu Tyr Trp Gly Pro Thr Pro Arg Thr

115                 120                 125
Thr Asp Lys Leu Ile Phe Gly Lys Gly Thr Arg Val Thr Val Glu Pro
    130                 135                 140
Arg Ser Gln Pro His Thr Lys Pro Ser Val Phe Val Met Lys Asn Gly
145                 150                 155                 160
Thr Asn Val Ala Cys Leu Val Lys Glu Phe Tyr Pro Lys Asp Ile Arg
                165                 170                 175
Ile Asn Leu Val Ser Ser Lys Lys Ile Thr Glu Phe Asp Pro Ala Ile
            180                 185                 190
Val Ile Ser Pro Ser Gly Lys Tyr Asn Ala Val Lys Leu Gly Lys Tyr
        195                 200                 205
Glu Asp Ser Asn Ser Val Thr Cys Ser Val Gln His Asp Asn Lys Thr
    210                 215                 220
Val His Ser Thr Asp Phe Glu Val Lys Thr Asp Ser Thr Asp His Val
225                 230                 235                 240
Lys Pro Lys Glu Thr Glu Asn Thr Lys Gln Pro Ser Lys Ser Cys His
                245                 250                 255
Lys Pro Lys Ala Ile Val His Thr Glu Lys Val Asn Met Met Ser Leu
            260                 265                 270
Thr Val Leu Gly Leu Arg Met Leu Phe Ala Lys Thr Val Ala Val Asn
        275                 280                 285
Phe Leu Leu Thr Ala Lys Leu Phe Phe Leu
    290                 295

<210> 17
<211> 303
<212> PRT
<213> Homo sapiens

<220>
<221> SIGNAL
<222> 1..20
<223> signal peptide for expression in insect cells

<220>
<223> TRD soluble (TCR04, aa)

<220>
<221> BINDING
<222> 267..303
<223> Twin-Strep-Tag

<400> 17
Met Lys Leu Cys Ile Leu Leu Ala Val Val Ala Phe Val Gly Leu Ser
1               5                   10                  15
Leu Gly Arg Ser Ala Gln Lys Val Thr Gln Ala Gln Ser Ser Val Ser
            20                  25                  30
Met Pro Val Arg Lys Ala Val Thr Leu Asn Cys Leu Tyr Glu Thr Ser
        35                  40                  45
Trp Trp Ser Tyr Tyr Ile Phe Trp Tyr Lys Gln Leu Pro Ser Lys Glu
    50                  55                  60
Met Ile Phe Leu Ile Arg Gln Gly Ser Asp Glu Gln Asn Ala Lys Ser
65                  70                  75                  80
Gly Arg Tyr Ser Val Asn Phe Lys Lys Ala Ala Lys Ser Val Ala Leu
                85                  90                  95
Thr Ile Ser Ala Leu Gln Leu Glu Asp Ser Ala Lys Tyr Phe Cys Ala
            100                 105                 110
Leu Gly Glu Leu Tyr Val Pro Leu Tyr Trp Gly Pro Thr Pro Arg Thr
        115                 120                 125
Thr Asp Lys Leu Ile Phe Gly Lys Gly Thr Arg Val Thr Val Glu Pro
    130                 135                 140
Arg Ser Gln Pro His Thr Lys Pro Ser Val Phe Val Met Lys Asn Gly

145                 150                 155                 160
Thr Asn Val Ala Cys Leu Val Lys Glu Phe Tyr Pro Lys Asp Ile Arg
                165                 170                 175
Ile Asn Leu Val Ser Ser Lys Lys Ile Thr Glu Phe Asp Pro Ala Ile
            180                 185                 190
Val Ile Ser Pro Ser Gly Lys Tyr Asn Ala Val Lys Leu Gly Lys Tyr
        195                 200                 205
Glu Asp Ser Asn Ser Val Thr Cys Ser Val Gln His Asp Asn Lys Thr
    210                 215                 220
Val His Ser Thr Asp Phe Glu Val Lys Thr Asp Ser Thr Asp His Val
225                 230                 235                 240
Lys Pro Lys Glu Thr Glu Asn Thr Lys Gln Pro Ser Lys Ser Cys His
                245                 250                 255
Lys Pro Lys Ala Ile Val His Thr Glu Lys Ser Arg Asp Asp Asp Asp
            260                 265                 270
Lys Ala Gly Trp Ser His Pro Gln Phe Glu Lys Gly Gly Gly Ser Gly
        275                 280                 285
Gly Gly Ser Gly Gly Gly Ser Trp Ser His Pro Gln Phe Glu Lys
    290                 295                 300

<210> 18
<211> 303
<212> PRT
<213> Homo sapiens


<220>
<221> SIGNAL
<222> 1..20
<223> signal peptide for expression in insect cells

<220>
<223> TRG soluble (aa)

<220>
<221> BINDING
<222> 267..303
<223> Twin-Strep-Tag

<400> 18
Met Lys Leu Cys Ile Leu Leu Ala Val Val Ala Phe Val Gly Leu Ser
1               5                   10                  15
Leu Gly Arg Ser Ser Ser Asn Leu Glu Gly Arg Thr Lys Ser Val Thr
            20                  25                  30
Arg Gln Thr Gly Ser Ser Ala Glu Ile Thr Cys Asp Leu Thr Val Thr
        35                  40                  45
Asn Thr Phe Tyr Ile His Trp Tyr Leu His Gln Glu Gly Lys Ala Pro
    50                  55                  60
Gln Arg Leu Leu Tyr Tyr Asp Val Ser Thr Ala Arg Asp Val Leu Glu
65                  70                  75                  80
Ser Gly Leu Ser Pro Gly Lys Tyr Tyr Thr His Thr Pro Arg Arg Trp
                85                  90                  95
Ser Trp Ile Leu Arg Leu Gln Asn Leu Ile Glu Asn Asp Ser Gly Val
            100                 105                 110
Tyr Tyr Cys Ala Thr Trp Asp Arg Gly Arg Lys Leu Phe Gly Ser Gly
        115                 120                 125
Thr Thr Leu Val Val Thr Asp Lys Gln Leu Asp Ala Asp Val Ser Pro
    130                 135                 140
Lys Pro Thr Ile Phe Leu Pro Ser Ile Ala Glu Thr Lys Leu Gln Lys
145                 150                 155                 160
Ala Gly Thr Tyr Leu Cys Leu Leu Glu Lys Phe Phe Pro Asp Val Ile
                165                 170                 175
Lys Ile His Trp Gln Glu Lys Lys Ser Asn Thr Ile Leu Gly Ser Gln

180                     185                     190
Glu Gly Asn Thr Met Lys Thr Asn Asp Thr Tyr Met Lys Phe Ser Trp
        195                     200                     205
Leu Thr Val Pro Glu Lys Ser Leu Asp Lys Glu His Arg Cys Ile Val
    210                     215                     220
Arg His Glu Asn Asn Lys Asn Gly Val Asp Gln Glu Ile Ile Phe Pro
225                     230                     235                     240
Pro Ile Lys Thr Asp Val Ile Thr Met Asp Pro Lys Asp Asn Cys Ser
                245                     250                     255
Lys Asp Ala Asn Asp Thr Leu Leu Leu Gln Ser Arg Asp Asp Asp Asp
                260                     265                     270
Lys Ala Gly Trp Ser His Pro Gln Phe Glu Lys Gly Gly Gly Ser Gly
        275                     280                     285
Gly Gly Ser Gly Gly Gly Ser Trp Ser His Pro Gln Phe Glu Lys
    290                     295                     300

<210> 19
<211> 25
<212> DNA
<213> Artificial Sequence

<220>
<223> V3-forward primer

<400> 19
gaagatcttc cagcaatctg gaggg                                          25

<210> 20
<211> 26
<212> DNA
<213> Artificial Sequence

<220>
<223> V3-reverse primer

<400> 20
gctctagact gcagcagcag ggtatc                                         26

<210> 21
<211> 27
<212> DNA
<213> Artificial Sequence

<220>
<223> V1-forward primer

<400> 21
gaagatctgc ccagaaggtg acccagg                                        27

<210> 22
<211> 29
<212> DNA
<213> Artificial Sequence

<220>
<223> V1-reverse primer

<400> 22
gctctagact tctctgtgtg cacgatggc                                          29


<210> 23
<211> 20
<212> DNA
<213> Artificial Sequence


<220>
<223> TRB KO in JE6.1 gRNA1

<400> 23
ggctcaaaca cagcgacctc                                                    20


<210> 24
<211> 20
<212> DNA
<213> Artificial Sequence


<220>
<223> TRB KO in JE6.1 gRNA2

<400> 24
ggctctcgga gaatgacgag                                                    20


<210> 25
<211> 20
<212> DNA
<213> Artificial Sequence


<220>
<223> HLA-DRA KO in Raji

<400> 25
tgcattggcc aacatagctg                                                    20


<210> 26
<211> 20
<212> DNA
<213> Artificial Sequence


<220>
<223> RFXAP KO in Raji

<400> 26
acaccgcaac aagatgtaca                                                    20


<210> 27
<211> 16
<212> PRT
<213> Homo sapiens

<220>
<223> CDR3 of TCR02

<400> 27
Ala Leu Gly Glu Leu Pro Arg His His Leu Thr Asp Lys Leu Ile Phe
1               5                   10                  15

<210> 28
<211> 14
<212> PRT
<213> Homo sapiens

<220>
<223> peptide loaded on HLA-DR - CLIP87-101

<400> 28
Pro Val Ser Lys Met Arg Met Ala Thr Pro Leu Leu Met Gln
1               5                   10

<210> 29
<211> 14
<212> PRT
<213> Homo sapiens

<220>
<223> peptide loaded on HLA-DR- CLIP87-101

<400> 29
Leu Pro Leu Lys Met Leu Asn Ile Pro Ser Ile Asn Val His
1               5                   10

<210> 30
<211> 16
<212> PRT
<213> Cytomegalovirus

<220>
<223> peptide loaded on HLA-DR - CMV pp65 116-129

<400> 30
Ala Cys Tyr Glu Phe Leu Trp Gly Pro Arg Ala Leu Val Glu Thr Ser
1               5                   10                  15

<210> 31
<211> 13
<212> PRT
<213> Homo sapiens

<220>
<223> peptide loaded on HLA-DR - MAGE-A3 267-282

<400> 31
Thr Ser Leu Tyr Asn Leu Arg Arg Gly Thr Ala Leu Ala
1               5                   10

<210> 32
<211> 11
<212> PRT
<213> Human herpesvirus 4

<220>
<223> peptide loaded on HLA-DR - EBV EBNA1 515-527

<400> 32
Thr Val Phe Tyr Asn Ile Pro Pro Met Pro Leu
1               5                   10

<210> 33
<211> 15
<212> PRT
<213> Human herpesvirus 4

<220>
<223> peptide loaded on HLA-DR - EBV EBNA2 280-290

<400> 33
Leu Arg Gln Met Arg Thr Val Thr Pro Ile Arg Met Gln Gly Gly
1               5                   10                  15

<210> 34
<211> 15
<212> PRT
<213> Dermatophagoides pteronyssinus

<220>
<223> peptide loaded on HLA-DR - house dust mit DR p1 96-110

<400> 34
Thr Arg Arg Gly Arg Val Lys Ile Asp Glu Val Ser Arg Met Phe
1               5                   10                  15

<210> 35
<211> 15
<212> PRT
<213> Cytomegalovirus

<220>
<223> peptide loaded on HLA-DR - HCMV IE2

<400> 35
Thr Arg Arg Gly Arg Val Lys Ile Asp Glu Val Ser Arg Met Phe
1               5                   10                  15

<210> 36
<211> 15
<212> PRT
<213> Cytomegalovirus

<220>
<223> peptide loaded on HLA-DR - HCMV IE2

<400> 36
Val Lys Gln Ile Lys Val Arg Val Asp Met Val Arg His Arg Ile

1                    5                        10                       15